# NEW EUROPEAN PATENT SPECIFICATION

(11) **EP 2 016 746 B2**
(45) Date of publication and mention of the opposition decision: **20.03.2019**
(45) Mention of the grant of the patent: 30.03.2016
(21) Application number: 07761393.3
(22) Date of filing: 26.04.2007
(51) Int. Cl.: H04L 29/08, A61B 5/00, A61M 5/172, A61M 5/145, G06F 19/00, A61B 5/145

(54) **ROUTER DEVICE AND DATA COMMUNICATION TECHNIQUES FOR NETWORKED FLUID INFUSION SYSTEMS**
ROUTERGERÄT UND DATENKOMMUNIKATIONSVERFAHREN FÜR VERNETZTE FLÜSSIGKEITSINFUSIONSSYSTEME
DISPOSITIF ROUTEUR ET TECHNIQUES DE COMMUNICATION DE DONNEES POUR SYSTEMES DE PERFUSION DE FLUIDES EN RESEAU

(30) Priority: 28.04.2006 US 413974; 18.10.2006 US 583344
(43) Date of publication of application: 21.01.2009
(73) Proprietor: Medtronic MiniMed, Inc., Northridge, CA 91325-1219 (US)
(72) Inventor: MOBERG, Sheldon B., Thousand Oaks, California 91362 (US); MEHTA, Kaezad J., Chatsworth, California 91311 (US); HANSON, Ian B., Granada Hills, CA 91344 (US); HOLTZCLAW, Kris R., Santa Clarita, California 91350 (US); LONG, Kenny J., Simi Valley, California 93063 (US)
(74) Representative: Ruschke, Hans Edvard
(86) International application number: PCT/US2007/067563
(87) International publication number: WO 2007/127880

(56) References cited:
- WO-A-2005/065538
- US-A1- 2002 013 518
- US-A1- 2004 102 683
- US-A1- 2004 263 354

## Description

### TECHNICAL FIELD

Embodiments of the system described herein relate generally to wireless telemetry for medical devices. More particularly, embodiments of the system described herein relate to a wireless router that provides centralized management, network control, and monitoring of patient and status information generated by various devices within a medical device system.

### BACKGROUND

Diabetics are usually required to modify and monitor their daily lifestyle to keep their body in balance, in particular, their blood glucose ("BG") levels. Individuals with Type 1 diabetes and some individuals with Type 2 diabetes use insulin to control their BG levels. To do so, diabetics routinely keep strict schedules, including ingesting timely nutritious meals, partaking in exercise, monitoring BG levels daily, and adjusting and administering insulin dosages accordingly.

The prior art includes a number of insulin pump systems that are designed to deliver accurate and measured doses of insulin via infusion sets (an infusion set delivers the insulin through a small diameter tube that terminates at a cannula inserted under the patient's skin). In lieu of a syringe, the patient can simply activate the insulin pump to administer an insulin bolus as needed, for example, in response to the patient's current BG level. A patient can measure his BG level using a BG measurement device, such as a test strip meter, a continuous glucose measurement system, or the like. BG measurement devices use various methods to measure the BG level of a patient, such as a sample of the patient's blood, a sensor in contact with a bodily fluid, an optical sensor, an enzymatic sensor, or a fluorescent sensor. When the BG measurement device has generated a BG measurement, the measurement is displayed on the BG measurement device. A continuous glucose monitoring system can monitor the patient's BG level in real time.

Insulin pumps and continuous glucose monitoring devices may also be configured to communicate with remote control devices, monitoring or display devices, BG meters, and other devices associated with such an infusion system. Individual devices within conventional infusion systems may be configured to support a limited amount of wired or wireless data communication to support the operation of the infusion system. For example, a continuous glucose monitoring sensor may include a wireless transmitter that communicates with a BG monitor device within the infusion system. As another example, the infusion system may include a handheld remote control that communicates with the infusion pump device using wireless techniques. Conventional infusion systems, however, operate in a somewhat isolated and local manner in that the routing of control signals, monitoring signals, patient status information, physiologic data, alerts, activation instructions, programming signals, and other data communication generally occurs within the limited short range and local operating environment of the infusion system itself.

### BRIEF SUMMARY

An embodiment of a medical device system as described here is suitably configured to communicate with one or more external network devices, such as networked computers, cellular telephones, personal digital assistants, hospital monitoring equipment, pager devices, or the like. Network communications from local devices within the medical device system may convey device status information, physiologic patient data, alerts, and/or alarms to the external devices. Such network communications may include notifications to third parties (parents, caregivers, medical equipment manufacturers) transmitted via email, pager messages, telephone calls, or any suitable data communication format. Moreover, network communications from external devices outside the local system environment may convey device programming instructions, device actuation instructions, calibration parameters, alert/alarm enable or disable signals, and/or other control parameters to the local system devices.

An embodiment of a network router device as described here is suitably configured to receive physiological sensor data from a plurality of medical devices (such as continuous glucose sensor transceivers worn by different patients). The network router device is also configured to communicate with one or more external network devices, such as networked computers, personal digital assistants, hospital monitoring equipment, or the like. The network router device facilitates centralized gathering, processing, storage, and formatting of the sensor data, and the network router device can be configured to generate web pages containing the sensor data and/or calibrated measurements based on the sensor data for remote web browser viewing.

The above and other aspects may be carried out by an embodiment of a monitor device for a medical device system. The monitor device comprises: a first communication module configured to receive a local communication from a transmitting device within the medical device system; a processing architecture coupled to the first communication module, the processing architecture being configured to interpret information conveyed in the local communication; a second communication module coupled to the processing architecture, the second communication module being configured to generate a network communication in response to the information; and a network interface coupled to the second communication module, the network interface enabling transmission of the network communication from the monitor device to a receiving device external to the medical device system.

The above and other aspects may also be carried out by an embodiment of a handheld monitor/controller device for a medical device system. The monitor device comprises: a first communication module configured to receive a local communication from a transmitting device within the medical device system; a processing architecture coupled to the first communication module, the processing architecture being configured to interpret information conveyed in the local communication; a second communication module coupled to the processing architecture, the second communication module being configured to generate a network communication in response to the information; and a wireless network interface coupled to the second communication module, the wireless network interface enabling wireless transmission of the network communication from the monitor device to a receiving device external to the medical device system.

The above and other aspects may also be carried out by an embodiment of a method for remote monitoring of an infusion system having an infusion pump that controls the infusion of fluid into the body of a user. The method comprises: receiving, at a network device that is external to the infusion system, a network communication generated by a transmitting device within the infusion system, the network communication conveying pump data associated with the infusion pump; extracting the pump data from the network communication; and generating, at the network device, indicia of the pump data.

The above and other aspects of the invention may also be carried out by an embodiment of a method for a medical device system. The method comprises: obtaining, at a transmitting device within the medical device system, a notification related to operation of a local device; generating a network communication in compliance with a network data communication protocol, the network communication conveying the notification; and transmitting, in accordance with the network data communication protocol, the network communication to a receiving device external to the medical device system.

The above and other aspects may also be carried out by an embodiment of a network-based medical device system. The system comprises: a monitor device for a medical device system, the monitor device comprising a communication module and a network interface coupled to the communication module, the communication module being configured to generate a network communication; and a network device external to the medical device system, the network device and the network interface being configured to enable transmission of the network communication from the monitor device to the network device via a network communication link.

The above and other aspects may also be carried out by an embodiment of a communication method for a wireless telemetry router device. The method comprises: receiving, at the wireless telemetry router device, a plurality of wireless communication signals, each of the wireless communication signals conveying sensor data generated by a respective physiological characteristic sensor; generating a network communication in compliance with a network data communication protocol, the network communication conveying at least some of the sensor data; and transmitting, in accordance with the network data communication protocol, the network communication to a network device.

The above and other aspects of the invention may also be carried out in one form by a data communication device comprising: a wireless communication module configured to support wireless data communication with a wireless medical device operating within a local system; a memory element coupled to the wireless communication module and configured to store data conveyed in wireless signals received from the wireless medical device; and a network interface coupled to the wireless communication module and configured to support transmission of network communications between the data communication device and a network device.

The above and other features may be implemented in one embodiment by a method for centralized processing of remote medical device data. The method involves: receiving, at a network router device, a communication signal that conveys a sensor measurement value, where the sensor measurement value is generated by a physiological characteristic sensor; obtaining, at the network router device, a patient calibration value corresponding to the sensor measurement value; and calculating, at the network router device, a calibrated physiological characteristic measurement value from the patient calibration value and the sensor measurement value.

The above and other features may also be implemented in an embodiment by a method for centralized processing of medical device data. The method involves: obtaining, at a network router device, a first sensor measurement value that corresponds to a first medical device; obtaining, at the network router device, a second sensor measurement value that corresponds to a second medical device; the network router device calculating a first calibrated physiological characteristic measurement value from the first sensor measurement value and a first patient calibration value; and the network router device calculating a second calibrated physiological characteristic measurement value from the second sensor measurement value and a second patient calibration value.

The above and other features may also be implemented in an embodiment by a network router device for centralized processing of medical device data. The network router device includes: a first data communication interface configured to receive a plurality of wireless communication signals, each of the wireless communication signals conveying a sensor measurement value generated by a respective physiological characteristic sensor; a processing architecture coupled to the first data communication interface, the processing architecture being configured to generate network communications in compliance with a network data communication protocol, the network communications conveying calibrated physiological characteristic measurement values corresponding to sensor measurement values; and a second data communication interface coupled to the processing architecture, the second data communication interface being configured to transmit, in accordance with the network data communication protocol, the network communications to at least one network device.

The above and other features may also be implemented in an embodiment by a network router device for centralized processing of medical device data. The network router device includes: a first data communication interface configured to receive a communication signal that conveys a sensor measurement value generated by a physiological characteristic sensor; a processing architecture coupled to the first data communication interface, the processing architecture being configured to calculate a calibrated physiological characteristic measurement value from the sensor measurement value and a patient calibration value; and a second data communication interface coupled to the processing architecture, the second data communication interface being configured to communicate the calibrated physiological characteristic measurement value to a network device that is coupled to the network router device.

This summary is provided to introduce a selection of concepts in a simplified form that are further described below in the detailed description. This summary is not intended to identify key features or essential features of the claimed subject matter, nor is it intended to be used as an aid in determining the scope of the claimed subject matter.

### BRIEF DESCRIPTION OF THE DRAWINGS

A more complete understanding of the present invention may be derived by referring to the detailed description and claims when considered in conjunction with the following figures, wherein like reference numbers refer to similar elements throughout the figures.
FIG. 1 is a schematic representation of a network-based infusion system configured in accordance with an example embodiment of the invention;
FIG. 2 is a front view of a bedside infusion system monitor configured in accordance with an example embodiment of the invention;
FIG. 3 is a front view of a hospital infusion system monitor configured in accordance with an example embodiment of the invention;
FIG. 4A is a front view of a handheld infusion system monitor/controller configured in accordance with example embodiment of the invention;
FIG. 4B is a front view of a handheld infusion system monitor/controller configured in accordance with another example embodiment of the invention;
FIG. 5 is a schematic representation of an infusion system monitor configured in accordance with an example embodiment of the invention;
FIG. 6 is a schematic representation of a network interface suitable for use with the infusion system monitor depicted in FIG. 5;
FIG. 7 is a schematic representation of a network communication module suitable for use with the infusion system monitor depicted in FIG. 5;
FIG. 8 is a schematic representation of a network-based infusion system configured in accordance with an example embodiment of the invention;
FIG. 9 is a flow chart that depicts an example network-based infusion system monitoring process;
FIG. 10 is a flow chart that depicts an example network-based infusion system communication process;
FIG. 11 is a flow chart that depicts an example network-based infusion pump monitoring and control process;
FIGS. 12-17 are screen shots that may be generated by monitor devices, controller devices, network devices, display devices, and/or other infusion system devices configured in accordance with example embodiments of the invention;
FIG. 18 is a perspective view of a data communication translation device configured in accordance with an example embodiment of the invention;
FIG. 19 is a schematic representation of a data communication translation device configured in accordance with an example embodiment of the invention;
FIG. 20 is a flow chart that depicts an example data storage and translation process;
FIG. 21 is a schematic representation of an example network deployment of a wireless telemetry router configured in accordance with an example embodiment of the invention;
FIG. 22 is a schematic representation of an example deployment of a network router device configured in accordance with another embodiment;
FIG. 23 is a schematic representation of a network router device configured in accordance with an embodiment of the invention;
FIG. 24 is a schematic representation of a processing architecture suitable for use with the network router device shown in FIG. 23;
FIG. 25 is a schematic representation of a memory element suitable for use with the network router device shown in FIG. 23;
FIG. 26 is a flow chart of a setup, management, and control process suitable for use with a network router device as described herein;
FIG. 27 is a flow chart of a data processing and routing process suitable for use with a network router device as described herein;
FIG. 28 is a sample screen shot of a patient monitor web page generated by a network router device as described herein; and
FIG. 29 is a sample screen shot of a patient reporting web page generated by a network router device as described herein.

### DETAILED DESCRIPTION

The following detailed description is merely illustrative in nature and is not intended to limit the embodiments of the invention or the application and uses of the embodiments of the invention. Furthermore, there is no intention to be bound by any expressed or implied theory presented in the preceding technical field, background, brief summary or the following detailed description.

Embodiments of the invention may be described here in terms of functional and/or logical block components and various processing steps. It should be appreciated that such block components may be realized by any number of hardware, software, and/or firmware components configured to perform the specified functions. For example, an embodiment of the invention may employ various integrated circuit components, e.g., memory elements, digital signal processing elements, logic elements, look-up tables, or the like, which may carry out a variety of functions under the control of one or more microprocessors or other control devices. In addition, those skilled in the art will appreciate that embodiments of the present invention may be practiced in conjunction with any number of data transmission protocols and that the system described here is merely one exemplary application for embodiments of the invention.

For the sake of brevity, conventional techniques related to infusion system operation, insulin pump and/or infusion set operation, blood glucose sensing and monitoring, signal processing, data transmission, signaling, network control, and other functional aspects of the systems (and the individual operating components of the systems) may not be described in detail here. Examples of infusion sets that may be used as a delivery device are described in, but not limited to, U.S. Patents: 4,723,947; 4,755,173; 5,176,662; 5,584,813; 6,056,718; 6,461,329; 6,475,195; 6,520,938; 6,585,695; 6,591,876; and 6,607,509, which are herein incorporated by reference. Examples of infusion pumps and/or communication options may be of the type described in, but not limited to, U.S. Patents: 4,562,751; 4,685,903; 5,080,653; 5,505,709; 5,097,122; 6,554,798; 6,558,320; 6,558,351; 6,641,533; 6,659,980; 6,752,787; 6,817,990; and 6,932,584, which are herein incorporated by reference. Examples of glucose sensing and/or monitoring devices maybe be of the type described in, but not limited to, U.S. Patents: 6,484,045; 6,809,653; 6,892,085; and 6,895,263, which are herein incorporated by reference. Furthermore, the connecting lines shown in the various figures contained here are intended to represent example functional relationships and/or physical couplings between the various elements. It should be noted that many alternative or additional functional relationships or physical connections may be present in an embodiment.

The following description may refer to elements or features being "connected" or "coupled" together. As used here, unless expressly stated otherwise, "connected" means that one element/feature is directly joined to (or directly communicates with) another element/feature, and not necessarily mechanically. Likewise, unless expressly stated otherwise, "coupled" means that one element/feature is directly or indirectly joined to (or directly or indirectly communicates with) another element/feature, and not necessarily mechanically. Thus, although each of the schematic block diagrams depicts one example arrangement of elements, additional intervening elements, devices, features, or components may be present in an embodiment (assuming that the functionality of the device or system is not adversely affected).

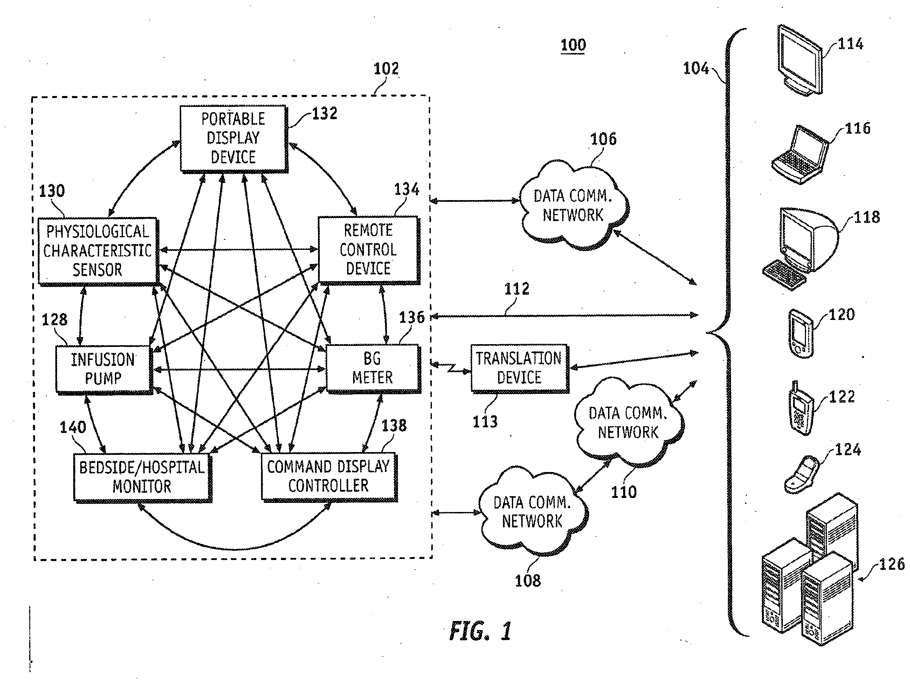

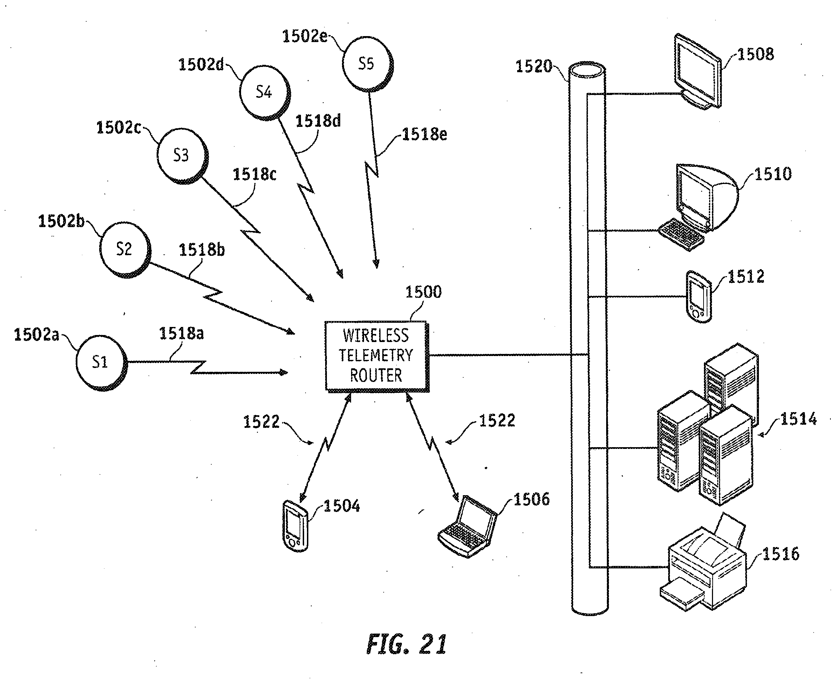

FIG. 1 is a schematic representation of a network-based medical device system 100 configured in accordance with an example embodiment of the invention. In this example, system 100 is an insulin infusion system that controls the infusion of insulin into the body of a user. Aspects of the invention, however, may also be utilized in the context of other medical device systems. Briefly, system 100 includes a local infusion system 102 having one or more local devices that communicate (unidirectional or bidirectional) with one or more network devices 104. As used here, network devices 104 are "external" to local infusion system 102 because they need not utilize the local data communication protocols and techniques employed within local infusion system 102, and because they need not be in close physical proximity to the local devices within local infusion system 102. The manner in which a given local device within local infusion system 102 communicates with a given network device 104 may vary depending upon the particular configuration of system 100, the characteristics of that local device, and the characteristics of that network device 104. For example, network communications may be routed using one data communication network 106, using a plurality of data communication networks 108/110, using a direct wireless or wired connection 112, or the like. In one example embodiment, data from wireless devices within local infusion system 102 (and/or data from wireless devices associated with different local infusion systems) may be collected by a wireless telemetry router device that serves as an interface to one or more network devices 104. One example wireless telemetry router device is described in more detail below in connection with FIG. 21.

Data communicated within local infusion system 102 and/or between devices within local infusion system 102 and network devices 104 may include or represent, without limitation: physiologic patient data, device status information, time and date information, alarm/alert status, and other information related to the operation, status, or condition of the patient, related to any of the devices within local infusion system 102, or related to local infusion system 102 itself. For example, such data may include or represent bolus information, basal information, or sensor information. Such data may also include or represent information entered by the patient, a caregiver, or another person having access to a local device or a network device 104, such as, without limitation: reminders; event markers (for meals, exercise, or the like); alarms; notifications; or the like.

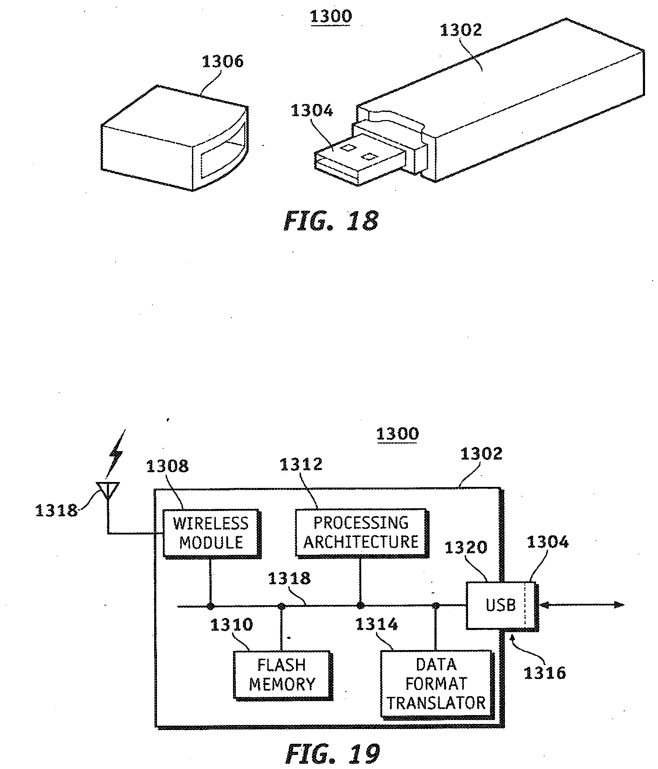

In one embodiment, devices within local infusion system 102 can communicate with network devices 104 via a suitably configured translation device, system, or application 113. For example, such a translation device 113 may be configured to communicate with devices within local infusion system 102 using a suitable RF data communication protocol (which may be published or proprietary), while coupling to one or more network devices 104 via a standardized data communication interface such as USB, IEEE 1394, or the like. The translation device 113 may also be provisioned with flash memory capability such that patients or caregivers can save data received from a device in a portable storage device and physically transport the storage device to any compatible computing device, e.g., a personal computer at a doctor's office. One example translation device is described in more detail below in connection with FIGS. 18-20.

As used here, a "data communication network" represents any number of physical, virtual, or logical components, including hardware, software, firmware, and/or processing logic configured to support data communication between an originating component and a destination component, where data communication is carried out in accordance with one or more designated communication protocols over one or more designated communication media. Communication hardware utilized by a data communication network may include a mechanically detachable unit such as an SDIO, a USB ready wireless module, or the like. For example, data communication network 106 may include, without limitation: a computer network such as a local area network or a wide area network; a pager network; a cellular telecommunication network; a cordless telephone system; an 802.11 network (WiFi); an 802.16 network (WiMAX); the Internet; IEEE P1901 BPL (Broadband over Power Lines); a hospital data communication network (WMTS or other); a home network, such as a home control network, a home security system, or a home alarm system; the public switched telephone network; a satellite communication network; or the like. In embodiments, network communications between local infusion system 102 and network devices 104 may be routed by two or more different types of data communication networks using known or proprietary network interfacing techniques.

The flexible nature of network-based infusion system 100 is illustrated in FIG. 1, which depicts local infusion system 102 in communication with a variety of external and remote network devices 104. In an embodiment, local devices within local infusion system 102 may be suitably configured to support the transmission of network communications to: a stationary monitor device 114, such as a bedside monitor or a piece of hospital monitoring equipment; a portable computer 116, such as a laptop PC, a palmtop PC, or a tablet PC; a stationary computer 118, such as a desktop PC; a personal digital assistant 120, which may also be a portable email device; a smart phone 122, which may also be a portable email device; a wireless phone 124, such as a cellular phone or a cordless phone; one or more additional computing devices or databases 126; or the like. As described in more detail below, these local devices need not communicate only via a local network interface and such devices may communicate using other means. The above list of possible network devices 104 is not exhaustive, and an implementation of system 100 can be designed to accommodate network communication with other network systems, equipment, computing devices, components, and elements that are external to local infusion system 102.

In one embodiment, local infusion system 102 is realized as an insulin infusion system that is locally controlled and monitored by the patient. In this example, local infusion system 102 includes at least an infusion pump 128. Local infusion system 102 may also include any of the following components, without limitation: a physiological characteristic sensor 130, such as a continuous glucose sensor (which may include a wireless transmitter); a portable display device 132; a remote control device 134; a BG meter 136 or other physiological characteristic meter; a command display controller 138 for infusion pump 128; and a monitor device 140, which may be realized as a bedside monitor or a hospital monitor. Each of these local devices is described in more detail below.

As depicted in FIG. 1, these local devices may be configured to transmit and receive local communications within local infusion system 102, where such local communications are transmitted and received in accordance with one or more specified local data communication protocols. For example, local communications may be exchanged between local devices using one or more wireless data communication protocols (which may leverage RF, infrared, magnetic induction, or other wireless techniques) and/or using one or more wired data communication protocols. Local infusion system 102 may be flexibly configured such that any given local device can communicate with any other local device, and a communication link or path between two local devices may be unidirectional or bidirectional. FIG. 1 depicts an example embodiment where each communication link or path is bidirectional (represented by double headed arrows).

Infusion pump 128 is configured to deliver fluid, such as insulin, into the body of a user via, for example, an infusion set. In accordance with one example embodiment, infusion pump 128 serves as a central hub, and most of the processing logic and intelligence for local infusion system resides at infusion pump 128. In some embodiments, the local medical device system need not include infusion pump 128, for example, monitoring systems utilized in conjunction with traditional insulin injection therapy. Moreover, infusion pump 128 need not include a display. In an embodiment that lacks a display, portable display device 132, remote control device 134, command display controller 138, or any other device within local infusion system 102 may serve as a remote display for infusion pump 128. Other options for a remote display include, but are not limited to, any of the network devices 104 described above, e.g., wireless phone 124, monitor device 114, portable computer 116, or personal digital assistant 120.

In practice, operation of infusion pump 128 may be remotely controlled by command display controller 138 (which may be realized as a handheld monitor/controller for infusion pump 128), by remote control device 134, and/or by or monitor 140. In one example embodiment, BG meter 136 may include the functionality of a controller device such that both components share a single housing. One such BG meter is described in U.S. patent application serial number 11/204,667, titled "Controller Device for an Infusion Pump," the content of which is incorporated by reference herein. Control of infusion pump 128 may also be possible via a suitably configured user interface located at infusion pump 128 itself.

Local infusion system 102 may also include physiologic characteristic sensor 130, which is suitably configured to measure a physiologic characteristic of the patient. In addition, sensor 130 may include processing and control logic that enables it to control the operation of infusion pump 128. Such control may be responsive to measurements obtained by sensor 130. In the example system described here, sensor 130 is a continuous BG sensor that measures the BG level of the patient in real time. Sensor 130 may include a wireless transmitter that facilitates transmission of physiologic data of the user to other devices within local infusion system 102. Alternatively, sensor 130 may be directly wired to a monitor/user interface. Sensor 130 may also be linked to monitor 140 so that monitoring and programming of medication delivery may be performed remotely. Alternatively sensor 130 may communicate directly with devices in the external network space, e.g., via Bluetooth, ZigBee or the like.

Local devices can process the received sensor data in an appropriate manner. For example, portable display device 132, remote control device 134, BG meter 136, command display controller 138, monitor 140, or infusion pump 128 may display the current BG level derived from the received sensor data and/or generate an alert or otherwise indicate low or high BG levels. As another example, BG meter 136 or infusion pump 128 may process the received sensor data for purposes of calibration. As yet another example, infusion pump 128 may be configured to activate its infusion mechanism in response to the received sensor data. Moreover, sensor data could be processed in one or more of the local devices and/or in one or more of network devices 104. In this regard, system 100 may utilize distributed processing techniques for the handling of sensor data.

Any of the devices within local infusion system 102 may include a display and related processing logic that facilitates the display of physiologic patient data, device status information, time and date information, alarm/alert status, and other information related to the operation, status, or condition of the patient, related to any of the devices within local infusion system 102, or related to local infusion system 102 itself. Portable display device 132 may be realized as a small device having limited functionality. In this regard, portable display device 132 may be incorporated into a key fob, a carabiner, a pendant, an insulin pen, a credit card display, or the like. Other local devices may have expanded display capabilities related to the specific functionality of such devices. For example, BG meter 136 may include display features that are specific to its metering functionality.

BG meter 136 is generally configured to measure the BG level of a user by analyzing a blood sample. For example, BG meter 136 may include a receptacle for receiving a blood sample test strip. In this regard, the user inserts a test strip into the BG meter 136, which analyzes the sample and displays a BG level corresponding to the test strip sample. BG meter 136 may be configured to generate a local communication, which conveys the measured BG level, for transmission to other local devices within local infusion system 102. Depending upon the specific application, BG meter 136 may also include the functionality of a monitoring device for infusion pump 128 and/or the functionality of a controller device for infusion pump 128.

Command display controller 138 is preferably realized as a handheld monitor/controller device that, although physically separate from infusion pump 128, enables the user to monitor and control the operation of infusion pump 128. This allows the user to operate infusion pump 128 without physically handling the device. As described in more detail below, command display controller 138 includes a communication module for transmitting local communications or commands to infusion pump 128. In further embodiments, command display controller 138 may receive local communications sent from infusion pump 128 or other components within local infusion system 102. In example embodiments, command display controller 138 also includes a network communication module for handling network communications to and from network devices that are external to local infusion system 102. Further, command display controller 138 may include one or more user input elements on its housing, such as keys, buttons, or the like, which accommodate user inputs. In embodiments, command display controller 138 includes a display on its housing, which may be configured to concurrently reproduce at least a portion of the information displayed on infusion pump 128.

Monitor 140, which may be realized as a bedside monitor for personal use or as a hospital monitor for caregiver use, enables remote monitoring of infusion pump 128 (and possibly other devices within local infusion system 102). Monitor 140 and other monitors described herein may be utilized in applications that do not utilize infusion pump 128; for example, applications that monitor patient data (such as glucose levels). In addition, monitor 140 may be suitably configured to enable remote programming and control of infusion pump 128 and/or other devices within local infusion system 102. In this regard, a "monitor" as used herein can generally refer to a monitor-only device or a monitor-controller device. In practice, monitor 140 is a relatively large device in comparison to portable or handheld devices of infusion system 102. In contrast to remote control device 134, portable display device 132, and command display controller 138, monitor 140 is intended to be somewhat stationary and not carried by the user. For example, a bedside monitor may be located on a nightstand beside the patient's bed, while a hospital monitor may be located on a medical equipment cart or stand in the patient's room. In contrast to the smaller portable devices of local infusion system 102, monitor 140 preferably includes a large and easy to read display element, which may be configured to concurrently reproduce at least a portion of the information displayed on infusion pump 128.

As described above in connection with command display controller 138, monitor 140 may also be configured to allow the user to remotely operate infusion pump 128. Monitor 140 may include a communication module for receiving and/or transmitting local communications within local infusion system 102. Moreover, monitor 140 may include a network communication module for handling network communications to and from network devices that are external to local infusion system 102. Further, monitor 140 may include one or more user input elements on its housing, such as keys, buttons, or the like, which accommodate user inputs.

As shown in FIG. 1, local infusion system 102 is capable of establishing many potential communication paths between the local devices. In embodiments, a controller device (e.g., remote control device 134, command display controller 138, or monitor 140) may serve as a translator between infusion pump 128 and the other components of local infusion system 102, such as BG meter 136. For example, the controller device may have the ability to determine how best to translate data received from infusion pump 128 for compatibility with the display requirements of a destination device within local infusion system 102. As depicted in FIG. 1, infusion pump 128 may communicate directly with BG meter 136. In some embodiments, local infusion system 102 may include multiple controllers that can communicate with infusion pump 128. In other embodiments, only one controller device can communicate with infusion pump 128 at any given moment. The controller device functionality may also be integrated into infusion pump 128 in some embodiments. In yet another embodiment, BG meter 136 may be integrated into the controller device such that both features share a single device housing.

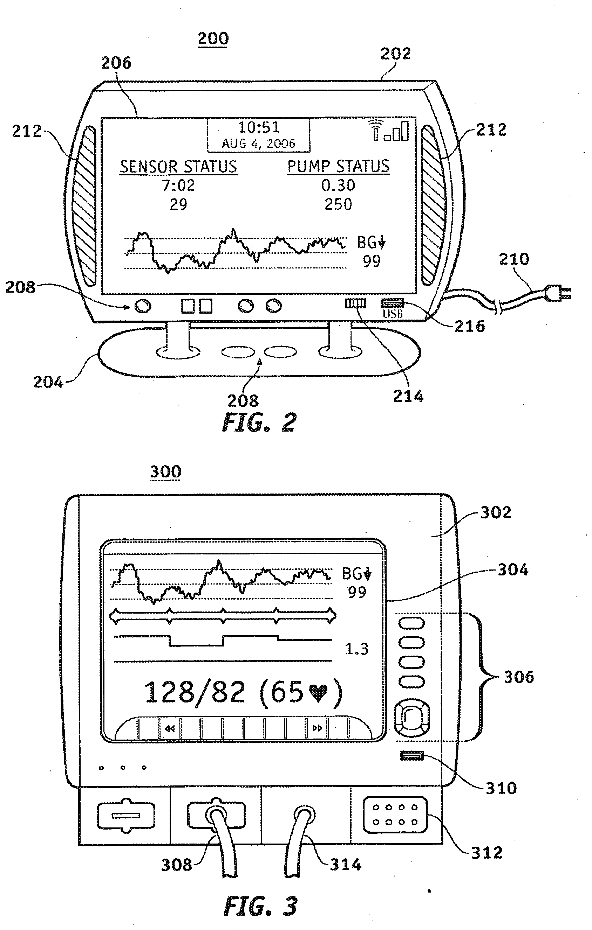

FIG. 2 is a front view of an example bedside monitor 200 configured in accordance with an example embodiment of the invention. Referring to FIG. 1, bedside monitor 200 may be deployed in local infusion system 102 (as monitor 140) and/or as a network device 104 (e.g., as monitor 114). Bedside monitor 200 may, but need not, be utilized to monitor the activity of an insulin infusion pump. Bedside monitor 200 generally includes a housing 202, a stand 204 that supports housing 202, a display element 206, and user interface features 208. Embodiments of bedside monitor 200 may include an AC power plug 210, one or more speakers 212, one or more local device interfaces 214, and one or more network interfaces 216.

As mentioned above, bedside monitor 200 is intended to be used as a somewhat stationary fixture placed in a suitable location, such as on the patient's nightstand. In other words, bedside monitor 200 is not designed to be a portable or handheld component. Therefore, housing 202 may be sized to accommodate a relatively large display element 206, which may utilize any known display technology (e.g., a cathode ray tube, an LCD panel, or a plasma panel). The size of display element 206 may vary to suit the needs of the particular application; typical sizes can range from 10 diagonal inches to 20 diagonal inches. Housing 202 may also be configured to accommodate integral speakers 212, which can be activated to generate alarm or alert notifications. Housing 202 may also be designed to accommodate user interface features 208 as shown in FIG. 2. Stand 204 is suitably configured to support housing 202 and to provide a stable mounting location for bedside monitor 200. In the example embodiment shown in FIG. 2, stand 204 is also configured to accommodate one or more user interface features 208. User interface features 208 may include a keypad, keys, buttons, switches, knobs, a touchpad, a joystick, a pointing device, a virtual writing tablet, or any device, component, or function that enables the user to select options, input information, or otherwise control the operation of bedside monitor 200.

Bedside monitor 200 may include processing logic, a display driver, and memory (not shown in FIG. 2) that is suitably configured to display information on display element 206. In embodiments, bedside monitor 200 functions to display information requested by the user, to display information related to an instructed act that was undertaken by the infusion pump, or to display status data for the infusion pump, such as, for example, BG levels, BG trends or graphs, or fluid delivery information. Bedside monitor 200 may be configured to display information conveyed in local communications received from an infusion pump or from any device within the local infusion system. At any moment, display element 206 may show substantially the same information as shown on the infusion pump; the two displays may mimic one another so that the user may choose to conveniently view the selected information from bedside monitor 200 rather than from the infusion pump, which is usually attached to the patient's body through an infusion set. Display element 206 may also include a backlight to facilitate viewing. The backlight may be a user programmable multicolor backlight that additionally performs the function of a visual indicator by flashing colors appropriate to the level of an alert or alarm. The backlight may also have variable intensity (automatic or manual) to accommodate user preferences and/or to indicate different alert or alarm status.

As described in more detail below, bedside monitor 200 may include one or more communication modules (not shown in FIG. 2) that facilitate data communication between bedside monitor 200 and other local devices within the local infusion system and/or data communication between bedside monitor 200 and network devices that are external to the local infusion system. For example, a local communication module may cooperate with a local device interface to receive local communications from local devices and/or to transmit local communications to local devices. The local communication module and local device interface may be configured to support wireless and/or wired data communication protocols. In an embodiment, local device interface 214 may represent a physical interface (such as a plug, a jack, a connector, a USB port, etc.) that facilitates connection to a data communication cable or any suitably configured physical component that establishes a communication link to a local device. As another example, a network communication module may cooperate with a network interface to receive network communications from network devices and/or to transmit network communications to network devices. The network communication module and network interface may be configured to support wireless and/or wired data communication protocols. In an embodiment, network interface 216 may represent a physical interface (such as a plug, a jack, a connector, a USB port, etc.) that accommodates a data communication cable or any suitably configured physical component that establishes a communication link to a network device. Bedside monitor 200 may also utilize one or more wireless local device interfaces and one or more wireless network interfaces, however, such wireless interfaces may not be visible from points outside housing 202.

FIG. 3 is a front view of an example hospital monitor 300 configured in accordance with an example embodiment of the invention. Hospital monitor 300 is similar to bedside monitor 200, and both monitors include some shared features and functionality. For the sake of brevity, such common features and functions will not be redundantly described here. Hospital monitor 300 is generally configured to display and/or process information in an appropriate manner. Such information may be, for example, alarms, alerts, or any of the information or data types described above with respect to FIG. 1, regardless of the location or device that originally generated or processed such information/data. Generally, referring to FIG. 1, hospital monitor 300 may be deployed in local infusion system 102 (as monitor 140) and/or as a network device 104 (e.g., as monitor 114). Hospital monitor 300 generally includes a housing 302, a display element 304, user interface features 306, an AC power plug 308, one or more speakers (hidden from view in FIG. 3), one or more local device interfaces 310, and one or more network interfaces 312. In this example embodiment, hospital monitor 300 also includes an integrated infusion pump that delivers fluid to the patient via a delivery tube 314.

Hospital monitor 300 is intended to be used as a somewhat stationary fixture placed in a suitable location, such as on a cart or an equipment rack in the patient's room. In other words, hospital monitor 300 is not designed to be a portable or handheld component. Hospital monitor 300 is suitably configured to operate substantially as described above with respect to bedside monitor 200. In contrast to bedside monitor 200, however, hospital monitor 300 may include an infusion pump and control features related to the operation of the infusion pump. Moreover, hospital monitor 300 may employ a network communication module and a network interface that cooperate to receive network communications from hospital network devices and/or to transmit network communications to hospital network devices. As used here, a "hospital network" refers to any number of physical or logical components, including hardware, software, firmware, and/or processing logic configured to support data communication between an originating component and a destination component, where data communication is carried out in accordance with one or more communication protocols that are reserved for, or utilized in, hospital environments.

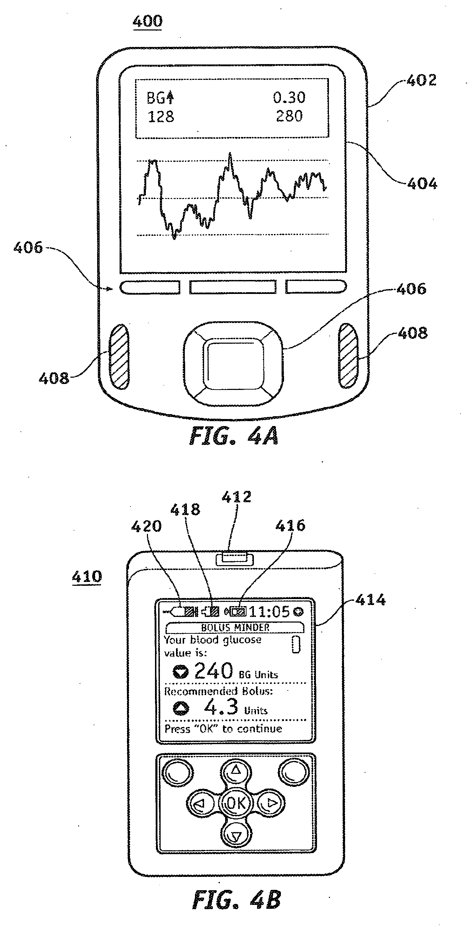

FIG. 4A is a front view of a handheld monitor/controller 400 configured in accordance with an example embodiment of the invention. Handheld monitor/controller 400 is similar to bedside monitor 200, and both monitors include some shared features and functionality. For the sake of brevity, such common features and functions will not be redundantly described here. Referring to FIG. 1, handheld monitor/controller 400 may be deployed in local infusion system 102 (as command display controller 138 or remote control device 134) and/or as a network device 104 (e.g., as personal digital assistant 120). Handheld monitor/controller 400 generally includes a housing 402, a display element 404, user interface features 406, one or more speakers 408, one or more local device interfaces (not shown), and one or more network interfaces (not shown).

Handheld monitor/controller 400 is intended to be used as a portable and mobile device that can be carried by the user. In particular embodiments, handheld monitor/controller 400 supports wireless communication with the patient's infusion pump, and the telemetry range of handheld monitor/controller 400 is localized. Handheld monitor/controller 400 is suitably configured to operate substantially as described above in connection with bedside monitor 200. Although the example embodiment utilizes a wireless local device interface and a wireless network interface, handheld monitor/controller 400 may also include wired interfaces to accommodate direct physical connections to other devices within the local infusion system and/or to network devices external to the local infusion system.

The power of handheld monitor/controller 400 (and of the other portable devices discussed here) may be provided by a battery. The battery may be a single use or a rechargeable battery. Where the battery is rechargeable, there may be a connector or other interface on handheld monitor/controller 400 for attaching the device to an electrical outlet, docking station, portable recharger, or so forth to recharge the battery while the battery remains in housing 402. It is also possible that a rechargeable battery may be removable from housing 402 for external recharging. In practice, however, the rechargeable battery may be sealed into housing 402 to create a more water resistant or waterproof component. In further embodiments, handheld monitor/controller 400 may be adapted to accommodate more than one type of battery. For example, handheld monitor/controller 400 may be configured to accommodate a rechargeable battery and (for backup or emergency purposes) a readily available battery type, such as a AA battery, a AAA battery, or a coin cell battery.

FIG. 4B is a front view of a handheld monitor/controller 410 configured in accordance with another example embodiment of the invention. Handheld monitor/controller 410 is similar to handheld monitor/controller 400, and both devices include some shared features and functionality. For the sake of brevity, such common features and functions will not be redundantly described here.

Handheld monitor/controller 410 preferably includes wireless data communication functionality that enables it to handle wireless local communications and/or wireless network communications. In addition, handheld monitor/controller 410 may include a wired or cabled network interface 412, which may be realized as a cable connector, jack, plug, or receptacle. FIG. 4B depicts example content displayed on a display element 414 of handheld monitor/controller 410. This content represents one particular "screen shot" for handheld monitor/controller 410; in practice any number of different display screens can be generated to suit the intended functionality and features of the device. The example screen shot of FIG. 4B includes a clock display, an RF quality indicator 416, a battery indicator 418, a fluid level indicator 420 that represents the amount of fluid remaining in the infusion pump, a current BG value for the patient (240 in this example), and a recommended bolus (4.3 units in this example). Handheld monitor/controller 410 may also display one or more prompts that provide guidance or instruction to the user. In this example, display element 414 includes the prompt: "Press 'OK' to Continue". The user can press "OK" to display other options, such as an activation request that controls the infusion pump to administer the recommended bolus.

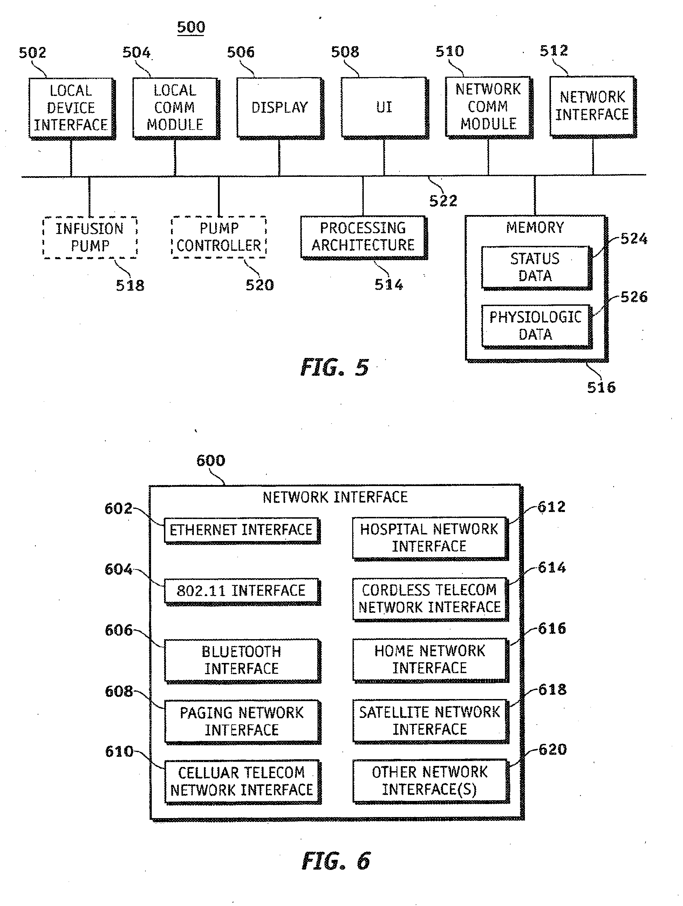

FIG. 5 is a schematic representation of a medical device system monitor 500 configured in accordance with an example embodiment of the invention. Monitor 500 represents a generalized embodiment that may be realized as a bedside monitor, a hospital monitor, or a handheld monitor/controller, depending upon its specific configuration. In this example, monitor 500 generally includes a local device interface 502, a local communication module 504, a display element 506, one or more user interface features 508, a network communication module 510, a network interface 512, a processing architecture 514, and a suitable amount of memory 516. If monitor 500 is implemented as a hospital monitor, then it may also include an infusion pump 518 and a pump controller 520 that controls the operation of infusion pump 518 (these elements are depicted in dashed lines to indicate their optional nature). The elements of monitor 500 may be coupled together via a bus 522 or any suitable interconnection architecture.

Those of skill in the art will understand that the various illustrative blocks, modules, circuits, and processing logic described in connection with monitor 500 (and other devices, elements, and components disclosed here) may be implemented in hardware, computer software, firmware, or any combination of these. To clearly illustrate this interchangeability and compatibility of hardware, firmware, and software, various illustrative components, blocks, modules, circuits, and processing steps may be described generally in terms of their functionality. Whether such functionality is implemented as hardware, firmware, or software depends upon the particular application and design constraints imposed on the embodiment. Those familiar with the concepts described here may implement such functionality in a suitable manner for each particular application, but such implementation decisions should not be interpreted as causing a departure from the scope of the present invention.

Referring again to FIG. 5, display element 506 and user interface features 508 were described above in connection with bedside monitor 200, hospital monitor 300, and handheld monitor/controller 400. Briefly, display element 506 is suitably configured to enable monitor 500 to display physiologic patient data, local device status information, clock information, alarms, alerts, and any information/data received or processed by monitor 500. For example, display element 506 may be controlled to indicate an alert or alarm status when monitor 500 receives an incoming communication (from a local device within the infusion system or from a network device external to the infusion system) that conveys an alert signal or an alarm signal. User interface features 508 enable the user to control the operation of monitor 500. In one example embodiment, user interface features 508 enable the user to control the operation of one or more additional devices within the local infusion system, for example, an infusion pump. Moreover, monitor 500 may be configured such that user interface features 508 can be manipulated to control the operation of one or more network devices that are external to the local infusion system.

Processing architecture 514 may be implemented or performed with a general purpose processor, a content addressable memory, a digital signal processor, an application specific integrated circuit, a field programmable gate array, any suitable programmable logic device, discrete gate or transistor logic, discrete hardware components, or any combination designed to perform the functions described here. A processor may be realized as a microprocessor, a controller, a microcontroller, or a state machine. Moreover, a processor may be implemented as a combination of computing devices, e.g., a combination of a digital signal processor and a microprocessor, a plurality of microprocessors, one or more microprocessors in conjunction with a digital signal processor core, or any other such configuration.

In practice, processing architecture 514 may be suitably configured to interpret and process incoming information, data, and content that is conveyed in local communications received from a transmitting device within the local infusion system. Referring to FIG. 1, the transmitting device may be any of the devices within local infusion system 102, including another monitor device. Such incoming information may include, without limitation: physiologic data of the user, such as a BG level (a calibrated reading or a raw measured value); status information of the transmitting local device (e.g., a battery life indication, a power on/off status, a transmit signal power level, diagnostic information indicating results of self tests); an alert signal related to operation of the transmitting local device (e.g., a low battery alert, an out of range alert, a calibration reminder); a basal rate of fluid delivered to the user by an infusion pump; bolus information for a bolus of fluid delivered to the user by an infusion pump; advisory information for the patient (e.g., a notification to place an order for supplies, a reminder to schedule a doctor's appointment, a reminder to schedule or automatically execute a data download for analysis by a caregiver, a notification to perform routine diagnostics, either manually or remotely via a network connection); or the like.

Processing architecture 514 may also be configured to interpret and process incoming information, data, and content that is conveyed in network communications generated by an originating device that is external to the local infusion system. Referring to FIG. 1, the originating device may be any network device 104, including a networked monitor device. Such incoming network information may include, without limitation: programming data for a local device within the infusion system; an activation instruction for an infusion pump or another local device within the infusion system; a status request for a local device within the infusion system; a request for physiologic data of the user; an alert or alarm enable or disable instruction for a local device within the infusion system (which may be processed by monitor 500 and/or routed by monitor 500 to the appropriate local device); advisory information for the patient (e.g., a notification to place an order for supplies, a reminder to schedule a doctor's appointment, a reminder to schedule or automatically execute a data download for analysis by a caregiver, a notification to perform routine diagnostics, either manually or remotely via a network connection); or the like.

Memory 516 may be realized as RAM memory, flash memory, EPROM memory, EEPROM memory, registers, a hard disk, a removable disk, a CD-ROM, or any other form of storage medium known in the art. In this regard, memory 516 can be coupled to processing architecture 514 such that processing architecture 514 can read information from, and write information to, memory 516. In the alternative, memory 516 may be integral to processing architecture 514. As an example, processing architecture 514 and memory 516 may reside in an ASIC. In this example, memory 516 may be utilized to store device status data 524 and/or physiologic data 526 of the user, where such data is communicated to monitor 500 via local communications, network communications, or directly (for example, if monitor 500 is configured to receive BG data directly from a test strip or via direct user input).

Monitor 500 may be configured to communicate with a remote database or databank that is accessible via a network connection. Referring to FIG. 1, for example, a network device 104 in system 100 may be realized as a network database 126 that provides data to monitor 500. In such an embodiment, monitor 500 can download data from the remote database as necessary, store it in memory 516 if needed, or otherwise process the downloaded data in an appropriate manner.

An embodiment of monitor 500 may employ any number of local communication modules 504 and any number of local device interfaces 502. For simplicity, the example described here employs one local communication module 504 and one local device interface 502. Local communication module 504 and local device interface 502 are suitably configured to support local communications between monitor 500 and devices within the local infusion system (e.g., any of the devices in infusion system 102 shown in FIG. 1). Depending upon the particular implementation, local communication module 504 and local device interface 502 may be configured to support unidirectional communication from monitor 500 to one or more local devices, unidirectional communication from one or more local devices to monitor 500, or bidirectional communication between monitor 500 and one or more local devices. Thus, local device interface 502 may be configured to receive a local communication from a transmitting device within the local infusion system, and/or to transmit a local communication to a receiving device within the local infusion system. Moreover, depending upon the particular implementation, local communication module 504 and local device interface 502 may be configured to support wireless data communication, wired/cabled data communication, or both.

For wireless transmissions of local communications, local communication module 504 and local device interface 502 support one or more wireless data communication protocols that are also supported by the local device(s) communicating with monitor 500. Any number of suitable wireless data communication protocols, techniques, or methodologies may be supported by monitor 500, including, without limitation: RF; IrDA (infrared); Bluetooth; ZigBee (and other variants of the IEEE 802.15 protocol); IEEE 802.11 (any variation); IEEE 802.16 (WiMAX or any other variation); Direct Sequence Spread Spectrum; Frequency Hopping Spread Spectrum; cellular/wireless/cordless telecommunication protocols; wireless home network communication protocols; paging network protocols; magnetic induction; satellite data communication protocols; wireless hospital or health care facility network protocols such as those operating in the WMTS bands; GPRS; and proprietary wireless data communication protocols such as variants of Wireless USB. In an embodiment, a wireless local device interface 502 may include or be realized as hardware, software, and/or firmware, such as an RF front end, a suitably configured radio module (which may be a stand alone module or integrated with other or all functions of the device), a wireless transmitter, a wireless receiver, a wireless transceiver, an infrared sensor, an electromagnetic transducer, or the like.

For transmissions of local communications over a cable, a wired connection, or other physical link, local communication module 504 and local device interface 502 support one or more wired/cabled data communication protocols that are also supported by the local device(s) communicating with monitor 500. Any number of suitable data communication protocols, techniques, or methodologies may be supported by monitor 500, including, without limitation: Ethernet; home network communication protocols; USB; IEEE 1394 (Firewire); hospital network communication protocols; and proprietary data communication protocols. In an embodiment, a wired/cabled local device interface 502 may include or be realized as hardware, software, and/or firmware, such as a suitably configured and formatted port, connector, jack, plug, receptacle, socket, adaptor, or the like.

An embodiment of monitor 500 may employ any number of network communication modules 510 and any number of network interfaces 512. For simplicity, the described example employs one network communication module 510 and one network interface 512. Network communication module 510 and network interface 512 are suitably configured to support network communications between monitor 500 and network devices that are external to the local infusion system (e.g., one or more of the network devices 104 shown in FIG. 1). Depending upon the particular implementation, network communication module 510 and network interface 512 may be configured to support unidirectional communication from monitor 500 to one or more network devices, unidirectional communication from one or more network devices to monitor 500, or bidirectional communication between monitor 500 and one or more network devices. Thus, network device interface 512 may be configured to receive an incoming network communication from an originating network device, and/or to enable transmission of an outgoing network communication to a receiving network device. Moreover, depending upon the particular implementation, network communication module 510 and network interface 512 may be configured to support wireless data communication, wired/cabled data communication, or both.

For wireless transmissions of network communications, network communication module 510 and network interface 512 support one or more wireless data communication protocols that are also supported by the network device(s) communicating with monitor 500. Any number of suitable wireless data communication protocols, techniques, or methodologies may be supported by monitor 500, including, without limitation, the wireless protocols listed above. In an embodiment, a wireless network interface 512 may include or be realized as hardware, software, and/or firmware, as described above for a wireless local device interface 502.

For transmissions of network communications over a cable, a wired connection, or other physical link, network communication module 510 and network interface 512 support one or more wired/cabled data communication protocols that are also supported by the network device(s) communicating with monitor 500. Any number of suitable data communication protocols, techniques, or methodologies may be supported by monitor 500, including, without limitation, the wired or cable based protocols listed above. In an embodiment, a wired/cabled network interface 512 may include or be realized as hardware, software, and/or firmware, as described above for a wired/cabled local device interface 502.

FIG. 6 is a schematic representation of a generalized network interface 600 suitable for use with monitor 500. For ease of description, network interface 600 is depicted as a general interface that includes a number of wireless and wired/cabled data communication aspects. Network interface 600 need not include multiple interfaces as depicted in FIG. 6 and, indeed, an embodiment may utilize only one specific type of interface. Network interface 600 generally includes an Ethernet interface 602, an 802.11 interface 604, a Bluetooth interface 606, a paging network interface 608, a cellular telecommunication network interface 610, a hospital network interface 612, a cordless telecommunication network interface 614, a home network interface 616, a satellite network interface 618, and other network interfaces 620.

Ethernet interface 602 may include or be realized as hardware, software, and/or firmware that is suitably configured to cooperate with network communication module 510 to accommodate Ethernet compliant network data communications with one or more network devices. For example, Ethernet interface 602 may include a T-568A Ethernet connector, a T-568B Ethernet connector, an RJ-45 connector, or any connector that is compatible with Ethernet cables.

802.11 interface 604 may include or be realized as hardware, software, and/or firmware that is suitably configured to cooperate with network communication module 510 to accommodate 802.11 compliant network data communications with one or more network devices. For example, 802.11 interface 604 may include an appropriate radio module, an 802.11 transceiver card, an RF front end, an RF antenna, and/or 802.11 access point functionality.

Bluetooth interface 606 may include or be realized as hardware, software, and/or firmware that is suitably configured to cooperate with network communication module 510 to support Bluetooth compliant network data communications with one or more network devices. For example, Bluetooth interface 606 may include an appropriate radio module, a Bluetooth transceiver, an RF front end, and/or an RF antenna.

Paging network interface 608 may include or be realized as hardware, software, and/or firmware that is suitably configured to cooperate with network communication module 510 to support network communications in compliance with a paging network protocol. For example, paging network interface 608 may include an appropriate radio module, a transceiver card, an RF front end, and/or an RF antenna.

Cellular telecommunication network interface 610 may include or be realized as hardware, software, and/or firmware that is suitably configured to cooperate with network communication module 510 to accommodate network communications in compliance with a cellular telecommunication protocol (e.g., CDMA, GSM, or the like). For example, cellular telecommunication network interface 610 may include an appropriate radio module, a transceiver card, an RF front end, and/or an RF antenna.

Hospital network interface 612 may include or be realized as hardware, software, and/or firmware that is suitably configured to cooperate with network communication module 510 to support network communications in compliance with a hospital network protocol. In embodiments, the hospital network protocol may be a wireless data communication protocol or a wired/cabled data communication protocol. In this regard, a wireless hospital network interface 612 may include an appropriate radio module, a transceiver card, an RF front end, an RF antenna, an infrared transmitter, an infrared sensor, a magnetic induction transducer, or the like. Depending upon the particular deployment, a wireless hospital network interface 612 may be compliant with any of the other wireless/cordless data communication protocols described here. A wired/cabled hospital network interface 612 may include suitably configured connectors, sockets, jacks, plugs, or adaptors. Moreover, depending upon the particular application, a wired/cabled hospital network interface 612 may be compliant with any of the other wired/cabled data communication protocols described here.

Cordless telecommunication network interface 614 may include or be realized as hardware, software, and/or firmware that is suitably configured to cooperate with network communication module 510 to support network communications in compliance with a cordless telecommunication protocol. Such protocols are commonly used in household cordless telephone systems. In practice, cordless telecommunication network interface 614 may include an appropriate radio module, a cordless telephone base station, a transceiver card, an RF front end, and/or an RF antenna.

Home network interface 616 may include or be realized as hardware, software, and/or firmware that is suitably configured to cooperate with network communication module 510 to support network communications in compliance with a home network protocol. Such home network protocols may be utilized in the context of a home control system, a home computing network that leverages existing telephone wires or existing AC power lines, a home security or alarm system, a home entertainment system, or the like. In embodiments, the home network protocol may be a wireless data communication protocol or a wired/cabled data communication protocol. In this regard, a wireless home network interface 616 may include an appropriate radio module, a transceiver base station, a transceiver card, an RF front end, an RF antenna, an infrared transmitter, an infrared sensor, a magnetic induction transducer, or the like. Depending upon the particular deployment, a wireless home network interface 616 may be compliant with any of the other wireless/cordless data communication protocols described here. A wired/cabled home network interface 616 may include suitably configured connectors, sockets, jacks, plugs, or adaptors. Moreover, depending upon the particular application, a wired/cabled home network interface 616 may be compliant with any of the other wired/cabled data communication protocols described here.

Satellite network interface 618 may include or be realized as hardware, software, and/or firmware that is suitably configured to cooperate with network communication module 510 to accommodate network communications in compliance with a satellite data communication protocol. For example, satellite network interface 618 may include an appropriate radio module, a transceiver card, an RF front end, and/or an RF antenna. Alternatively (or additionally), satellite network interface 618 may include suitably configured connectors, sockets, jacks, plugs, or adaptors that facilitate wired/cabled connection to a separate piece of satellite network equipment, e.g., a satellite dish or a satellite transceiver module.

In practice, network interface 600 may utilize any number of network interfaces 620 other than the specific types described above. Such other network interfaces 620 can be suitably configured to support network communications in accordance with existing data communication protocols, whether publicly known or proprietary. Moreover, other network interfaces 620 enable network interface 600 to support wireless or wired data communication protocols that may be developed in the future.

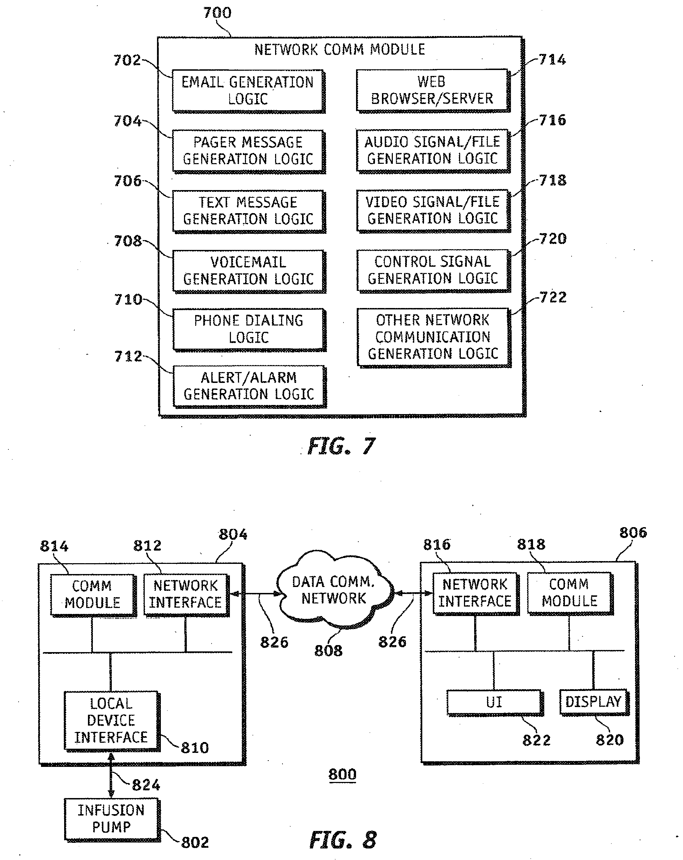

FIG. 7 is a schematic representation of a network communication module 700 suitable for use with monitor 500. For ease of description, network communication module 700 is depicted as a general module that includes processing logic for handling different types of network communications. In practice, network communication module 700 need not support different modes of network communications as depicted in FIG. 7 and, indeed, an embodiment may process only one specific network communication format or type. Network communication module 700 generally includes email generation logic 702, pager message generation logic 704, text message generation logic 706, voicemail generation logic 708, phone dialing logic 710, alert/alarm generation logic 712, a web browser/server 714, audio signal/file generation logic 716, video signal/file generation logic 718, control signal generation logic 720, and other network communication generation logic 722.

Email generation logic 702 may include or be realized as hardware, software, and/or firmware that is suitably configured to generate network communications as email. For example, email generation logic 702 may generate automatic or user-created email that conveys notifications, alerts, alarms, status reports, physiologic data, or other information that is intended for a destination network device. In embodiments, email generation logic 702 may be compatible with any suitable email system or technology, including web-based email systems.

Pager message generation logic 704 may include or be realized as hardware, software, and/or firmware that is suitably configured to generate network communications as pager messages. For example, pager message generation logic 704 may generate automatic or user-created pager messages that convey notifications, alerts, alarms, status reports, physiologic data, or other information that is intended for a pager device or any compatible destination network device. In embodiments, pager message generation logic 704 may be compatible with any suitable pager system or technology, including web-based paging systems.

Text message generation logic 706 may include or be realized as hardware, software, and/or firmware that is suitably configured to generate network communications as text messages. Such text messages may be carried over existing cellular telephone networks, existing pager networks, the Internet, local area networks, hospital networks, home networks, or the like. For example, text message generation logic 706 may generate automatic or user-created text messages that convey notifications, alerts, alarms, status reports, physiologic data, or other information that is intended for any compatible destination network device. In embodiments, text message generation logic 706 may be compatible with any suitable text messaging application or technology.

Voicemail generation logic 708 may include or be realized as hardware, software, and/or firmware that is suitably configured to generate network communications as voicemail messages. For example, voicemail message generation logic 708 may generate automatic or user-created voicemail messages that convey notifications, alerts, alarms, status reports, physiologic data, or other information that is intended for any compatible destination network device. In embodiments, such voicemail messages can be generated as audio files suitable for transmission as electronic attachments. Upon receipt, the destination network device can play the voicemail message using an appropriate playback mechanism, multimedia application, or the like. In embodiments, voicemail generation logic 708 may be compatible with any suitable voice messaging, telephone system, or multimedia application.

Phone dialing logic 710 may include or be realized as hardware, software, and/or firmware that is suitably configured to generate network communications as an outgoing telephone call. For example, phone dialing logic 710 may be configured to dial (automatically or in response to user interaction) an outgoing telephone number as needed to convey notifications, alerts, alarms, status reports, physiologic data, or other information that is intended for any compatible destination network device. Phone dialing logic 710 may also cooperate with one or more of the other logical components of network communication module 700, for example, voicemail generation logic 708, to facilitate transmission of certain network communications. In embodiments, phone dialing logic 710 may be compatible with any suitable telephone system or application.

Alert/alarm generation logic 712 may include or be realized as hardware, software, and/or firmware that is suitably configured to generate alerts and/or alarms intended for distribution to network devices. For example, alert/alarm generation logic 712 may generate automatic or user-created alerts or alarms that indicate any of the following, without limitation: battery status of a device within the local infusion system; when a physiologic characteristic of the patient crosses a predetermined threshold value; when a telemetered device within the local infusion system is out of range of the monitor; a scheduled calibration for a piece of equipment within the local infusion system; or any scheduled event related to the operation of the infusion system. In embodiments, alert/alarm generation logic 712 may cooperate with one or more of the other logical components of network communication module 700, for example, text message generation logic 706, to facilitate the formatting and network transmission of alerts and alarms. Upon receipt, the destination network device can generate an alert/alarm using an appropriate playback mechanism, multimedia application, an illuminating element, a speaker, or the like.

Web browser/server 714 represents a software application that is configured to generate network communications as markup language documents, e.g., HTML documents. Moreover, web browser/server 714 may include conventional web browsing capabilities that enable the monitor device to access web pages via the Internet. In this regard, web browser/server 714 may cooperate with one or more of the other logical components of network communication module 700, for example, email generation logic 702 or text message generation logic 706, to facilitate the transmission and receipt of certain network communications. Web browser applications and web server applications are well known and, therefore, will not be described in detail here.

Audio signal/file generation logic 716 may include or be realized as hardware, software, and/or firmware that is suitably configured to generate network communications as audio signals and/or audio files. The audio signals or files may be pre-programmed into the monitor device (or into the device that creates the audio signals or files). Alternatively, the audio signals or files may be created by a user of the monitor device (or by a user of the device in communication with the monitor device). For example, audio signal/file generation logic 716 may generate automatic or user-created audio signals or audio files that convey notifications, alerts, alarms, status reports, physiologic data, or other information that is intended for any compatible destination network device. Audio-based alerts/alarms may be automatically initiated by the monitor device or by a device in communication with the monitor device. Alternatively, audio-based alerts/alarms may be initiated by a user, patient, or caregiver at the monitor device or at a device in communication with the monitor device. Upon receipt, the destination network device can play the audio signals or audio files using an appropriate playback mechanism, multimedia application, or the like.

As used here, an audio signal may be a streaming audio signal, a broadcast radio signal, or a control signal that initiates the generation of audio at the destination network device, while an audio file represents a file that is received and interpreted by the destination network device (which then executes the audio file to generate audio). For example, audio signal/file generation logic 716 may be configured to generate MP3 audio files, WMA audio files, or the like. In this regard, audio signal/file generation logic 716 may cooperate with one or more of the other logical components of network communication module 700, for example, voicemail generation logic 708 or alert/alarm generation logic 712, to facilitate the transmission and receipt of certain network communications.

Video signal/file generation logic 718 may include or be realized as hardware, software, and/or firmware that is suitably configured to generate network communications as video signals and/or video files. The video signals or files may be pre-programmed into the monitor device (or into the device that creates the audio signals or files). Alternatively, the video signals or files may be created by a user of the monitor device (or by a user of the device in communication with the monitor device). For example, video signal/file generation logic 718 may generate automatic or user-created video signals or video files that convey notifications, alerts, alarms, status reports, physiologic data, or other information that is intended for any compatible destination network device. Video-based alerts/alarms may be automatically initiated by the monitor device or by a device in communication with the monitor device. Alternatively, video-based alerts/alarms may be initiated by a user, patient, or caregiver at the monitor device or at a device in communication with the monitor device. Upon receipt, the destination network device can play the video signals or video files using an appropriate playback mechanism, multimedia application, or the like.

As used here, a video signal may be a streaming video signal, a broadcast video signal, or a control signal that initiates the generation of video at the destination network device, while a video file represents a file that is received and interpreted by the destination network device (which then executes the video file to generate video). For example, video signal/file generation logic 718 may be configured to generate MPEG video files, JPG image files, or the like. In this regard, video signal/file generation logic 718 may cooperate with one or more of the other logical components of network communication module 700, for example, alert/alarm generation logic 712, to facilitate the transmission and receipt of certain network communications.

Control signal generation logic 720 may include or be realized as hardware, software, and/or firmware that is suitably configured to generate network communications as control signals for the receiving network device. For example, control signal generation logic 720 may generate automatic or user-created control signals that initiate the generation of notifications, alerts, alarms, displays, or otherwise control the operation of any compatible destination network device. Upon receipt of such a control signal, a destination network device will respond in a suitable manner - activating a display, activating a vibrating element, activating an illumination element, generating an audio or video response, or the like. In embodiments, control signal generation logic 720 may cooperate with one or more of the other logical components of network communication module 700, for example, alert/alarm generation logic 712, to facilitate the formatting and network transmission of control signals.

In practice, network communication module 700 may utilize other network communication generation logic 722 in lieu of, or in addition to, the specific types described above. Such other logical components can be suitably configured to generate network communications in various existing formats, whether publicly known or proprietary. Moreover, such other logical components enable network communication module 700 to support additional formats that may be developed in the future.

FIG. 8 is a schematic representation of a network-based medical device system 800 configured in accordance with an example embodiment of the invention. System 800 represents one simple implementation of a system that might utilize some of the devices, techniques, and methodologies described here. A vast number of alternative configurations may be constructed and operated within the scope of the invention. For example, although system 800 is described below in the context of an infusion pump, the infusion pump is not a requirement for embodiments of the invention.

Network-based infusion system 800 generally includes an infusion pump 802, a monitor device 804 (or any suitable local device that is defined to be within a local infusion system), and a network device 806. In this example embodiment, monitor device 804 and network device 806 communicate with each other via any number of network communication links established in a data communication network 808. Moreover, although not a requirement, FIG. 8 depicts bidirectional communications between monitor device 804 and network device 806. Network device 806 may be, for example, a network-based monitor, a networked computer, a cellular telephone or other mobile computing device, any network device 104 described in connection with FIG. 1, or any network-based device described elsewhere. Data communication network 808 may be (or include), for example, the Internet, a cellular telecommunication network, a paging system network, a local or wide area network, any wireless or wired network described in connection with FIG. 1, or any network described elsewhere.

As described in more detail in connection with FIG. 5, monitor 804 may include a local device interface 810, a network interface 812, and one or more suitable communication modules 814 (e.g., a local communication module and/or a network communication module). Network device 806 may include a network interface 816, which is configured for compatibility with network interface 812, one or more suitably configured communication modules 818, a display element 820, and user interface features 822. Network interface 816 may be configured as described above in connection with network interface 512 and in connection with network interface 600. Communication module(s) 818 may be configured as described above in connection with network communication module 510 and in connection with network communication module 700. Communication module(s) 818 are configured to enable network device 806 to receive, process, and interpret network communications received from monitor device 804. In addition, communication module(s) 818 may be configured to enable network device 806 to process, generate, and transmit outgoing network communications intended for monitor device 804. User interface features 822 and display element 820 enable a user of network device 806 to remotely view data that might be displayed at infusion pump 802 or monitor device 804, remotely control monitor device 804 or infusion pump 802, and/or remotely program or modify operating parameters of monitor device 804 or infusion pump 802.

In some embodiments of network-based infusion system 800, infusion pump 802 and monitor device 804 communicate using a first data communication protocol, while monitor device 804 and network device 806 communicate using a second data communication protocol (or a combination of protocols). Local communications between infusion pump 802 and monitor device 804 are carried over one or more local communication links 824, which may be wireless or wired. Network communications between monitor device 804 and network device 806 are carried over one or more network communication links 826, which may be wireless or wired. For example, infusion pump 802 may transmit local communications (such as pump status information) to monitor device 804, where the local communications are transmitted in accordance with a Bluetooth data communication protocol. Moreover, infusion pump 802 may receive incoming data from monitor device 804 using the same Bluetooth protocol. In contrast, monitor device 804 may transmit network communications (such as pump status information, alerts, or patient data) to network device 806, where the network communications are transmitted in accordance with a cellular telecommunication protocol such as CDMA. Similarly, monitor device 804 may receive incoming data from network device 806 using the same CDMA protocol.

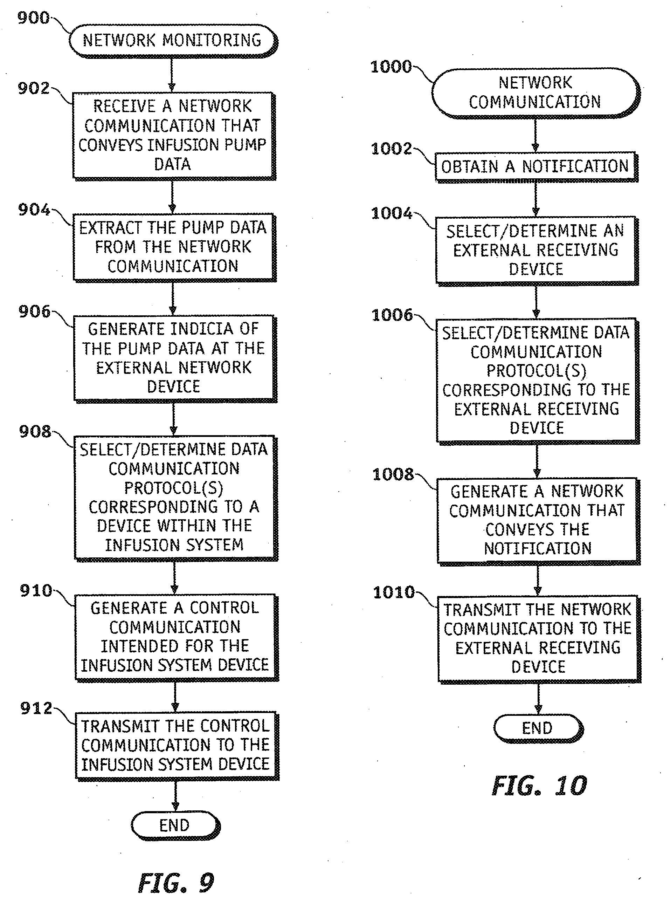

FIG. 9 is a flow chart that depicts an example network-based medical device system monitoring process 900. The various tasks performed in connection with process 900 may be performed by software, hardware, firmware, or any combination. For illustrative purposes, the following description of process 900 may refer to elements mentioned above in connection with FIGS. 1-8. In embodiments, portions of process 900 may be performed by different elements of the described system, e.g., a network device or a functional element or operating component. It should be appreciated that process 900 may include any number of additional or alternative tasks, the tasks shown in FIG. 9 need not be performed in the illustrated order, and process 900 may be incorporated into a more comprehensive procedure or process having additional functionality not described in detail here.

Monitoring process 900 may be performed by a network device that is external to a local infusion system having an infusion pump that controls the infusion of fluid into the body of a user. Process 900 may begin when the network device receives (task 902) a network communication that conveys pump data associated with the local infusion pump. The network communication may be generated by (or originate at) any transmitting device within the local infusion system, such as a bedside monitor device, a hospital monitor device, a physiological characteristic meter, a remote controller, a handheld monitor/controller, the infusion pump itself, or the like. The pump data may include any information or content related to the operation, control, programming, or status of the infusion pump and/or the transmitting device, including, without limitation: physiologic data of the user/patient, alarms, alerts, graph or chart data, a basal rate of fluid delivered by the infusion pump, bolus information for a bolus of fluid delivered by the infusion pump, or any suitably formatted text, audio, or visual information. As described above in connection with FIG. 5 and FIG. 6, the network device may receive the network communication in compliance with one or more appropriate data communication protocols, including, without limitation: an Ethernet protocol, an IEEE 802.11 protocol (any variant), a Bluetooth protocol, a paging network protocol, a cellular telecommunication protocol (e.g., CDMA or GSM), a cordless telecommunication protocol, a home network data communication protocol, a satellite data communication protocol, a hospital network protocol, or any suitable wireless or wired/cabled data communication protocol that enables the network device to receive network communications via a wireless, cabled, and/or wired communication link.

In practice, the network device processes the received network communication and extracts (task 904) the pump data from the network communication. Task 904 may be performed by a suitably configured communication module and/or a suitably configured processing architecture resident at the network device. In response to such processing, the network device may generate (task 906) indicia of the pump data for display, playback, broadcast, or rendering at the network device. In connection with task 906, the network device may: generate indicia of received physiologic data; generate indicia of local device status information; generate indicia of an alert or an alarm; generate indicia of a basal rate of fluid delivery; generate indicia of bolus information; or the like. In embodiments, the network device may generate indicia of the pump data in any suitable manner, including, without limitation: generating an audible representation of the pump data, such as an audible alarm, alert, recording, or audio signal; generating a visual representation of the pump data, such as a graph or a text display; activating an illumination element of the network device, e.g., an indicator light or a flashing display screen; or activating a vibration element of the network device.

Monitoring process 900 assumes that the network device can transmit network communications back to a device within the local infusion system. In this regard, process 900 may select or determine (task 908) one or more data communication protocols corresponding to a local device within the infusion system. Task 908 may be performed to ensure that the network device utilizes an appropriate protocol for compatible communication with the local device. The network device may also obtain or generate an instruction or programming parameter intended for the infusion pump or another local device within the infusion system. Such instructions or programming parameters may be generated by the network device or obtained from an operator of the network device. The network device may be configured to generate (task 910) a suitably configured control communication that conveys the instruction or programming parameter. Depending upon the particular system deployment and the specific operating conditions, an example control communication may include, without limitation: an alert disable instruction; an activation instruction for the infusion pump or any local device; a programming parameter for the infusion pump or any local device; or the upload of software programs (main application code or auxiliary function code such as motor control, RF telemetry code, or the like). Eventually, the network device can transmit (task 912) the control communication in an appropriate format and in compliance with the particular data communication protocol utilized for the communication session with the local device. Upon receipt, the receiving local device can process the control communication in an appropriate manner.

In alternate embodiments of the invention, monitoring process 900 can be modified for use in connection with a medical device system that does not include an infusion pump. For example, the tasks of process 900 may be performed in an equivalent manner to receive and process a network communication that conveys patient data, monitor data, or other medical device information that might originate at a device within the local system, and such data need not include pump data.

FIG. 10 is a flow chart that depicts an example network-based medical device system communication process 1000. The various tasks performed in connection with process 1000 may be performed by software, hardware, firmware, or any combination of these. For illustrative purposes, the following description of process 1000 may refer to elements mentioned above in connection with FIGS. 1-8. In embodiments, portions of process 1000 may be performed by different elements of the described system, e.g., a local device within an infusion system or a functional element or operating component. It should be appreciated that process 1000 may include any number of additional or alternative tasks, the tasks shown in FIG. 10 need not be performed in the illustrated order, and process 1000 may be incorporated into a more comprehensive procedure or process having additional functionality not described in detail here.

Network communication process 1000 may be performed by a transmitting device that is within a local medical device system, e.g., an infusion system having an infusion pump that controls the infusion of fluid into the body of a user. For example, the transmitting device may be any local device within the local infusion system, such as a bedside monitor device, a hospital monitor device, a physiological characteristic meter, a physiological characteristic sensor transmitter, a remote controller, a handheld monitor/controller, the infusion pump itself, or the like. Process 1000 may begin when the transmitting device obtains (either internally, from another device, or from a user) or generates a notification (task 1002) related to the operation of the infusion pump and/or related to the operation of another local device. As used here, a notification may be any signal, alert, alarm, content, data, or information that is intended to be forwarded to another device, or is utilized as a prompt or a trigger to invoke a response by the transmitting device.

Network communication process 1000 may select or determine (task 1004) an external receiving device, which will be a network device in this example, that represents the intended recipient of the notification. In addition, process 1000 may select or determine (task 1006) one or more data communication protocols corresponding to the intended external receiving device. Task 1006 may be performed to ensure that the local transmitting device utilizes an appropriate protocol for compatible communication with the network device. As described above in connection with FIG. 5 and FIG. 6, the local device may transmit network communications in compliance with one or more appropriate data communication protocols, including, without limitation: an Ethernet protocol, an IEEE 802.11 protocol (any variant), a Bluetooth protocol, a paging network protocol, a cellular telecommunication protocol (e.g., CDMA or GSM), a cordless telecommunication protocol, a home network data communication protocol, a satellite data communication protocol, a hospital network protocol, or any suitable wireless or wired/cabled data communication protocol that enables the local device to transmit network communications via a wireless, cabled, and/or wired communication link.

The local transmitting device may then generate (task 1008) a network communication that conveys the notification, where the network communication is compatible with the selected data communication protocol. In accordance with embodiments, the network communication may include any information or content related to the operation, control, programming, or status of the infusion pump and/or the transmitting device, including, without limitation: physiologic data of the user/patient, alarms, alerts, graph or chart data, a basal rate of fluid delivered by the infusion pump, bolus information for a bolus of fluid delivered by the infusion pump, or any suitably formatted text, audio, or visual information. As described above in connection with FIG. 7, the network communication may be formatted as (or include) different message types, file types, or signal types, including, without limitation: an email message; a pager message; a text message; a voicemail message; an outgoing telephone call to the receiving network device; a markup language document, such as a web page; an audio signal; an audio file; a video signal; or a video file.

Eventually, the local transmitting device transmits (task 1010) the network communication to the external receiving device. The local device transmits the network communication in accordance with the network data communication protocol selected during task 1006. In one example, the network communication is conveyed in an outgoing telephone call, and the local transmitting devices transmits the network communication by initiating an outgoing telephone call to the destination network device. In other example embodiments, task 1010 represents the transmission of a message, file, and/or signal having a specified type and format. Upon receipt of the network communication, the destination network device can process the notification in an appropriate manner.

In alternate embodiments of the invention, process 1000 can be modified for use in connection with a medical device system that does not include an infusion pump. For example, the tasks of process 1000 may be performed in an equivalent manner to process and transmit a network communication that conveys patient data, monitor data, or other medical device information that might originate at a device within the local system, and such information need not include pump data

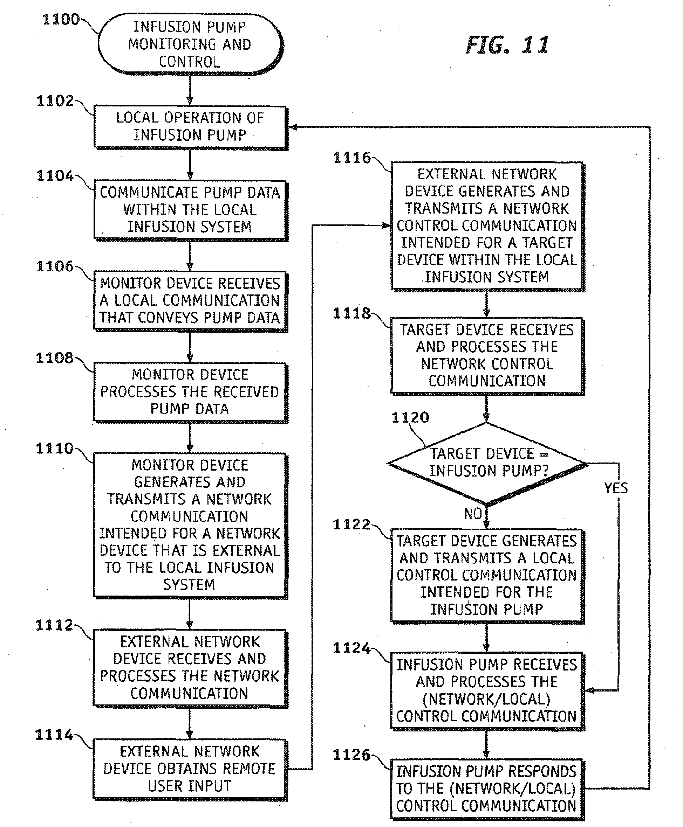

FIG. 11 is a flow chart that depicts an example network-based infusion pump monitoring and control process 1100. Process 1100 represents one example technique for operating a network-based infusion pump system. A system may be able to support any number of alternative techniques and methodologies, and the following description of process 1100 is not intended to limit the scope or application of the invention in any way. The various tasks performed in connection with process 1100 may be performed by software, hardware, firmware, or any combination. For illustrative purposes, the following description of process 1100 may refer to elements mentioned above in connection with FIGS. 1-8. In embodiments, portions of process 1100 may be performed by different elements of the described system, e.g., a local device, an infusion pump, a network device or any functional element or operating component. It should be appreciated that process 1100 may include any number of additional or alternative tasks, the tasks shown in FIG. 11 need not be performed in the illustrated order, and process 1100 may be incorporated into a more comprehensive procedure or process having additional functionality not described in detail here.

Infusion pump monitoring and control process 1100 is performed in conjunction with the normal local operation of an infusion pump (task 1102). Process 1100 preferably supports the communication of pump data within the local infusion system (task 1104), as described in detail above. In particular, task 1104 may correspond to the transmission of pump data from the infusion pump to a monitor device within the local infusion system, the transmission of pump data between local devices other than the infusion pump, or the like. In this example, a local monitor device receives a local communication that conveys pump data (task 1106). The local monitor device may be a bedside monitor, a hospital monitor, a handheld monitor/controller, or any suitably configured local device as described above. If necessary, the local monitor device processes the received pump data (task 1108) to determine how best to respond.

In this example, the local monitor device generates and transmits a network communication in response to the received pump data (task 1110). The network communication may be intended for any compatible network device that is external to the local infusion system. As described above, the network communication is preferably generated in accordance with a selected network data communication protocol that is also supported by the destination network device. Infusion pump monitoring and control process 1100 assumes that the external network device receives and processes (task 1112) the network communication in an appropriate manner. For example, the network device may generate an alert or an alarm that originated at the infusion pump.

In response to the network communication (e.g., an alert in this example), the network device may obtain a remote user input (task 1114). In this regard, a remote user input may correspond to manipulation of user interface features located at the network device. For example, the user of the network device may elect to disable the alert by engaging a "DISABLE" button on the network device. As another example, the user of the network device may elect to remotely administer a bolus by engaging an "ACTIVATE" button on the network device. In response to the remote user input, the network device may generate and transmit (task 1116) a suitably configured network control communication that is intended for a target device within the local infusion system. This control communication is formatted for compliance with a particular data communication protocol that is also supported by the target device. The target device may, but need not be, the same local device that transmitted (or originated) the local communication received during task 1106.

Infusion pump monitoring and control process 1100 assumes that the intended target device receives and processes (task 1118) the network control communication in an appropriate manner. Generally, the target device processes the received control communication to determine how best to respond. If the target device is the infusion pump, then process 1100 may proceed to a task 1124. If not, then process 1100 may proceed to a task 1122. During task 1122, the target device may generate and transmit a local control communication that is intended for the infusion pump. The target device generates and transmits the local control communication in accordance with a data communication protocol that is supported within the local infusion system. As an example, task 1122 can be performed when the target device is a local monitor device that locally communicates with the infusion device. Eventually, the infusion pump receives and processes (task 1124) the network or local control communication in an appropriate manner. In this regard, task 1124 is performed in response to the remote user input obtained at the network device during task 1114. In embodiments, the local infusion pump will respond to the control communication (task 1126) in a suitable manner. For example, the infusion pump may react in the following manner, without limitation: disable an alarm or an alert; update its software or firmware; modify its basal rate; activate its pump to administer a bolus; generate a local alert/alarm; perform a calibration routine; or the like.

In this example embodiment, infusion pump monitoring and control process 1100 enables continuous or periodic monitoring and control of the infusion pump. Accordingly, FIG. 11 depicts process 1100 as a loop, where task 1126 leads back to task 1102 for purposes of continued local operation of the infusion pump.

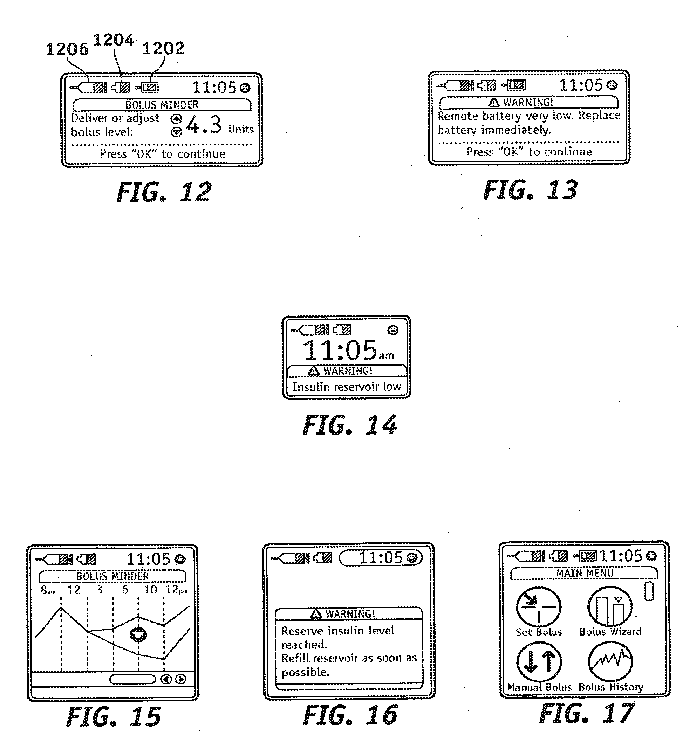

FIGS. 12-17 are screen shots that may be generated by monitor devices, controller devices, network devices, display devices, and/or other infusion system devices configured in accordance with example embodiments of the invention. For example, the content of these screen shots may be displayed by bedside monitor 200 (see FIG. 2), by hospital monitor 300 (see FIG. 3), by handheld monitor/controllers 400 and 410 (see FIG. 4), by any of the local devices within local infusion system 102 (see FIG. 1), and/or by any of the network devices 104 utilized by network-based infusion system 100 (see FIG. 1).

FIG. 12 is a screen shot that is suitable for use with a relatively small device, such as a handheld monitor, a personal digital assistant, a wireless phone, a key fob remote control, or the like. This screen shot includes a clock display, an RF quality indicator 1202, a battery indicator 1204, a fluid level indicator 1206 that represents the amount of fluid remaining in the infusion pump, and a recommended bolus (4.3 units in this example). This screen shot also includes the prompt: "Press 'OK' to Continue". The user can press "OK" to display other options, such as an activation request that controls the infusion pump to administer the recommended bolus.

FIG. 13 is another screen shot that is suitable for use with a relatively small device. This screen shot includes a warning display, which may be accompanied by a suitably generated alert or alarm. Here, the warning includes text that indicates a low battery condition and a reminder to replace the battery. In example embodiments of the invention, such a warning may be associated with the battery in the device that actually displays the warning, or it may be associated with the battery in a remote device being monitored by the device that actually displays the warning. In this regard, this screen shot may be displayed at a network monitor device, where the low battery warning indicates that the battery in the local infusion pump device is low.

FIG. 14 is a screen shot that is suitable for use with a small form factor device, such as a remote control, a watch sized monitor, a portable display-only device, or the like. This screen shot includes a clock display, which is proportionately large for readability. This screen shot also includes a warning display, which may be accompanied by a suitably generated alert or alarm. Here, the warning includes text that indicates a low insulin reservoir condition for the monitored infusion pump. In example embodiments, this screen shot can be displayed on the infusion pump itself, on a remote device within the local infusion system, and/or on a network-based monitoring device.

FIGS. 15-17 are various screen shots that are suitable for use with a relatively small device, such as a personal digital assistant, a wireless phone, or a pager device. The example screen shot of FIG. 15 includes historical BG data for the patient, rendered in a graph format, and a clock display. The screen shot of FIG. 16 includes a warning related to a low level in the insulin reservoir of the insulin pump, along with a clock display. The screen shot of FIG. 17 represents a "Main Menu" display for the device, where the menu includes a number of options for the user. For example, the device may display selectable menu icons, including, without limitation: a "Set Bolus" icon; a "Bolus Wizard" icon; a "Manual Bolus" icon; and a "Bolus History" icon. Selection of a given icon may cause the device to generate a new display screen that provides additional information or options related to the selected feature or function. For example, the "Set Bolus" icon enables the user to program the device for a specific bolus value or values that can be activated during use; the default values could be assigned to correspond to various meal carbohydrate values commonly consumed by the user, the "Bolus Wizard" icon launches a feature that enables the user to calculate a bolus of insulin that is appropriate for the patient's current condition, the "Manual Bolus" icon enables the user to deviate from the default bolus value(s), and the "Bolus History" icon launches a display (such as a graph, a chart, or a report) of past bolus deliveries by the infusion pump.

Again, the specific display formats, screen shot contents, display menu trees, and other display characteristics and features may vary depending upon the particular device configuration, whether the device is a network device or a local device within the infusion system, and/or whether the device is a wireless device. The example screen shots depicted in the various figures are not intended to limit or restrict the scope or application of any embodiment of the invention.

As mentioned above with regard to network-based infusion system 100 (see FIG. 1), a data communication translation device 113 may be utilized to facilitate communication between a wireless local device and a network device 104, such as a personal computer, a networked hospital computer, a caregiver office computer, or the like. FIG. 18 is a perspective view of a data communication translation device 1300 configured in accordance with one possible embodiment of the invention. In this embodiment, translation device 1300 is a relatively small and portable device that provides wireless bridge and memory storage functionality. Translation device 1300 may be conveniently sized such that it can be easily carried by a patient or a caregiver. In certain embodiments, translation device 1300 is small enough to be carried in a pocket.

Translation device 1300 includes a housing 1302 that encloses a number of functional components that are described in more detail below. This example embodiment includes a universal serial bus ("USB") connector 1304 that serves as a network interface port for translation device 1300. The network interface port can alternately be a IEEE 1394 port, a serial port, a parallel port, or the like. USB connector 1304 is configured for physical and electrical compliance with known USB specifications; such specifications will not be described in detail herein. Alternate embodiments may utilize different network interface configurations and, therefore, different network interface connectors, ports, couplers, or the like. USB connector 1304 is merely one suitable implementation of such a network interface, and embodiments of the invention are not limited to USB deployments.

Translation device 1300 may also include a removable cover 1306 that protects USB connector 1304 when translation device 1300 is not connected to a network device. Cover 1306 may be designed to snap onto USB connector 1304 and/or housing 1302 in a manner that allows the user to remove and replace cover 1306 by hand.

FIG. 19 is a schematic representation of one example embodiment of translation device 1300. In this example, translation device 1300 generally includes housing 1302, a network interface port (e.g., USB connector 1304), a wireless communication module 1308, a memory element 1310, a processing architecture 1312, a data format translator 1314, and a network interface 1316 (e.g., a USB interface). The elements of translation device 1300 may be coupled together via a bus 1318 or any suitable interconnection architecture. In example embodiments, housing 1302 encloses wireless communication module 1308, memory element 1310, processing architecture 1312, and data format translator 1314. Depending upon the particular implementation, housing 1302 may also enclose at least a portion of network interface 1316.

Processing architecture 1312 may be implemented or performed with a general purpose processor, a content addressable memory, a digital signal processor, an application specific integrated circuit, a field programmable gate array, any suitable programmable logic device, discrete gate or transistor logic, discrete hardware components, or any combination designed to perform the functions described here. A processor may be realized as a microprocessor, a controller, a microcontroller, or a state machine. Moreover, a processor may be implemented as a combination of computing devices, e.g., a combination of a digital signal processor and a microprocessor, a plurality of microprocessors, one or more microprocessors in conjunction with a digital signal processor core, or any other such configuration. In an example embodiment of translation device 1300, data format translator 1314 may be implemented in processing architecture 1312 (even though FIG. 19 depicts the two as separate logical elements).

In practice, processing architecture 1312 is configured to support the various tasks, functions, and operations of translation device 1300. For example, processing architecture 1312 may be suitably configured to interpret and process incoming information, data, and content that is conveyed in local communications received from a transmitting device within the local infusion system. Likewise, processing architecture 1312 may be suitably configured to interpret and process incoming information, data, and content that is conveyed in network communications received from a network device external to the local infusion system. Processing architecture 1312 may also be configured to manage storage and retrieval of data in memory element 1310. Moreover, processing architecture 1312 may be configured to process data in response to instructions received from a network device via network interface 1316 and/or in response to instructions received from a local device via wireless communication module 1308.

In one embodiment, memory element 1310 can be a powered memory arrangement that utilizes a backup battery to maintain its storage ability. In the example embodiment, memory element 1310 is realized as nonvolatile flash memory having a suitable amount of storage capacity. The design and configuration of flash memory, its selection circuitry, and its program/erase control circuitry are generally known, and such conventional aspects of memory element 1310 will not be described in detail here. In alternate embodiments, memory element 1310 may utilize EEPROM memory, random access memory, registers, a small scale hard disk, a removable media, or the like. In this regard, memory element 1310 can be coupled to processing architecture 1312 such that processing architecture 1312 can read information from, and write information to, memory element 1310. In the alternative, memory element 1312 and processing architecture 1312 may be realized as an integrated unit. As an example, processing architecture 1312 and memory element 1310 may reside in an ASIC. As described in more detail below, memory element 1310 can be utilized to store data conveyed in wireless signals received from a local device within an infusion system. In addition, memory element 1310 can be utilized to store data conveyed in network communication signals received from a network device external to the infusion system. Such data may include local device status data, physiologic data of the user, sensor data, alerts/alarms, control data from the network device, operating instructions for translation device 1300, any of the local data types or content described herein, and/or any of the network data types or content described herein.

Wireless communication module 1308 is suitably configured to support wireless data communication with a device within an infusion system, e.g., any of the local devices mentioned in the above description of infusion system 100 (see FIG. 1). For example, the local device may be an infusion pump or a monitor device for an infusion pump. Depending upon the particular implementation, wireless communication module 1308 may be configured to support unidirectional communication from local devices, or bidirectional communication between translation device 1300 and local devices. Thus, wireless communication module 1308 may be configured to receive local communication signals from a transmitting device within the local infusion system, and/or to transmit local communication signals to a receiving device within the local infusion system.

Wireless communication module 1308 may include or be realized as a radio module that supports one or more wireless data communication protocols and one or more wireless data transmission schemes. In an embodiment, wireless communication module 1308 may include or be realized as hardware, software, and/or firmware, such as an RF front end, a suitably configured radio module (which may be a stand alone module or integrated with other or all functions of translation device 1300), a wireless transmitter, a wireless receiver, a wireless transceiver, an infrared sensor, an electromagnetic transducer, or the like. In this example, translation device 1300 includes an antenna 1318 coupled to wireless communication module 1308. Antenna 1318, which may be located inside or outside of housing 1302 (or partially inside and partially outside of housing 1302), is appropriately configured in accordance with the particular design of wireless communication module 1308.

For wireless transmissions of local communications, wireless communication module 1308 supports one or more wireless data communication protocols that are also supported by the local device(s) communicating with translation device 1300. Any number of suitable wireless data communication protocols, techniques, or methodologies may be supported by wireless communication module 1308 and translation device 1300, including, without limitation: RF; IrDA (infrared); Bluetooth; ZigBee (and other variants of the IEEE 802.15 protocol); IEEE 802.11 (any variation); IEEE 802.16 (WiMAX or any other variation); Direct Sequence Spread Spectrum; Frequency Hopping Spread Spectrum; cellular/wireless/cordless telecommunication protocols; wireless home network communication protocols; paging network protocols; magnetic induction; satellite data communication protocols; wireless hospital or health care facility network protocols such as those operating in the WMTS bands; GPRS; and proprietary wireless data communication protocols such as variants of Wireless USB.

Network interface 1316 is generally configured to support transmission of network communications between translation device 1300 and one or more network devices. Network interface 1316 may include interface logic 1320 and network interface port 1304. Interface logic 1320 may be implemented in processing architecture 1312 (even though FIG. 19 depicts the two as separate logical elements). In this example embodiment, network interface 1316 is a USB interface, interface logic 1320 is compatible with USB specifications and requirements, and network interface port 1304 is a USB port or connector. As mentioned above, however, alternate embodiments may utilize different network interface configurations (for example, IEEE 1394) and, therefore, different network interface connectors, ports, couplers, or the like.

Network interface 1316 is suitably configured to support data communication with a device external to the infusion system, e.g., any of the network devices 104 mentioned in the above description of infusion system 100 (see FIG. 1). For example, the network device may be a personal computer having a suitable host application that can be manipulated to manage communication with translation device 1300. The personal computer may be owned by the patient, located in a caregiver facility, located in a hospital, located in a device manufacturer facility, or elsewhere. In example embodiments, the host application may be realized as software that is designed to provide monitoring, diagnostic services, patient data analysis, medical device programming, and/or other functions associated with one or more devices within the local infusion system. Depending upon the particular implementation, network interface 1316 may be configured to support unidirectional communication from translation device 1300, or bidirectional communication between translation device 1300 and network devices. Thus, network interface 1316 may be configured to receive network communication signals from a transmitting network device, and/or to transmit network communication signals to a receiving network device.

For transmission of network communication signals over a cable, a wired connection, a direct connection, or other physical link, network interface 1316 supports one or more wired/cabled data communication protocols that are also supported by the network device(s) communicating with translation device 1300. Any number of suitable data communication protocols, techniques, or methodologies may be supported by network interface 1316 and translation device 1300, including, without limitation: Ethernet; home network communication protocols; USB; IEEE 1394 (Firewire); hospital network communication protocols; and proprietary data communication protocols.

For wireless transmission of network communication signals, network interface 1316 supports one or more wireless data communication protocols that are also supported by the network device(s) communicating with translation device 1300. Any number of suitable wireless data communication protocols, techniques, or methodologies may be supported by network interface 1316 and translation device 1300, including, without limitation: RF; IrDA (infrared); Bluetooth; ZigBee (and other variants of the IEEE 802.15 protocol); IEEE 802.11 (any variation); IEEE 802.16 (WiMAX or any other variation); Direct Sequence Spread Spectrum; Frequency Hopping Spread Spectrum; cellular/wireless/cordless telecommunication protocols; wireless home network communication protocols; paging network protocols; magnetic induction; satellite data communication protocols; wireless hospital or health care facility network protocols such as those operating in the WMTS bands; GPRS; and proprietary wireless data communication protocols such as variants of Wireless USB.

In connection with wireless data transmissions, translation device 1300 may be configured to perform dynamic frequency hopping to optimize its operation, to conserve battery life for battery-powered wireless devices, and/or to provide flexibility in the complexity of the devices with which it communicates. For example, wireless communication module 1308 may be designed to dynamically accommodate 5-channel (low power) devices and 50-channel (high power) devices. In this context, translation device 1300 may utilize a low power mode to conserve battery power when a high quality wireless link has been established. On the other hand, translation device 1300 may switch to a high power mode in response to increased packet loss, increased collision, or a generally poor quality of service.

In connection with wireless data transmissions, translation device 1300 may also be configured to support a retry periodicity for synchronous links having a designated transmission periodicity. For example, during normal operation, a synchronous wireless link may communicate one packet per minute. Translation device 1300 can be configured to initiate a retry procedure in response to a missed packet. In this regard, translation device 1300 can support retry transmissions (i.e., retransmission of the missed packet) that occur at a higher rate than the normal operating mode. For example, retry, packet transmissions may occur every 20 seconds rather than once a minute. In practice, translation device 1300 and the wireless device may adapt their frequency hopping scheme to accommodate the retry packets, and resume their normal frequency hopping scheme thereafter.

Data format translator 1314, which may be realized as hardware, software, firmware, or any combination thereof, is suitably configured to reformat data between wireless communication module 1308 and network interface 1316. Depending upon the particular implementation, such reformatting may occur for data received via wireless communication module 1308, for data received via network interface 1316, or both. For example, it may be desirable for translation device 1300 to receive a wireless communication signal at wireless communication module 1308, extract data from the wireless communication signal, and process the extracted data in an appropriate manner such that the extracted data can be conveyed in a network communication signal to be provided by network interface 1316. Likewise, it may be desirable for translation device 1300 to receive a network communication signal at network interface 1316, extract data from the network communication signal, and process the extracted data in an appropriate manner such that the extracted data can be conveyed in a wireless communication signal to be provided by wireless communication module 1308.

Translation device 1300 may be configured to encrypt data between wireless communication module 1308 and network interface 1316. Encrypting data may be desirable for ensure that confidential or sensitive information remains protected. In this example, data format translator 1314 may be configured to perform data encryption using one or more known or proprietary encryption schemes. Alternatively, translation device 1300 may include a separate encryption engine or module that performs the data encryption. Depending upon the specific implementation, data encryption may be applied to the extracted data (or any portion thereof), to the sensitive/confidential data (or any portion thereof), and/or to the entire communication signal (or any portion thereof).

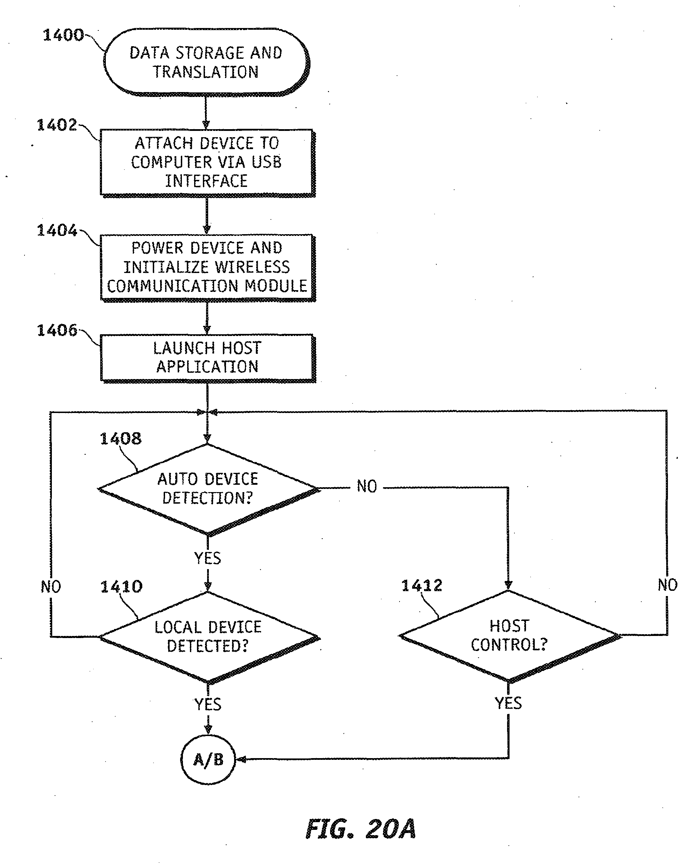

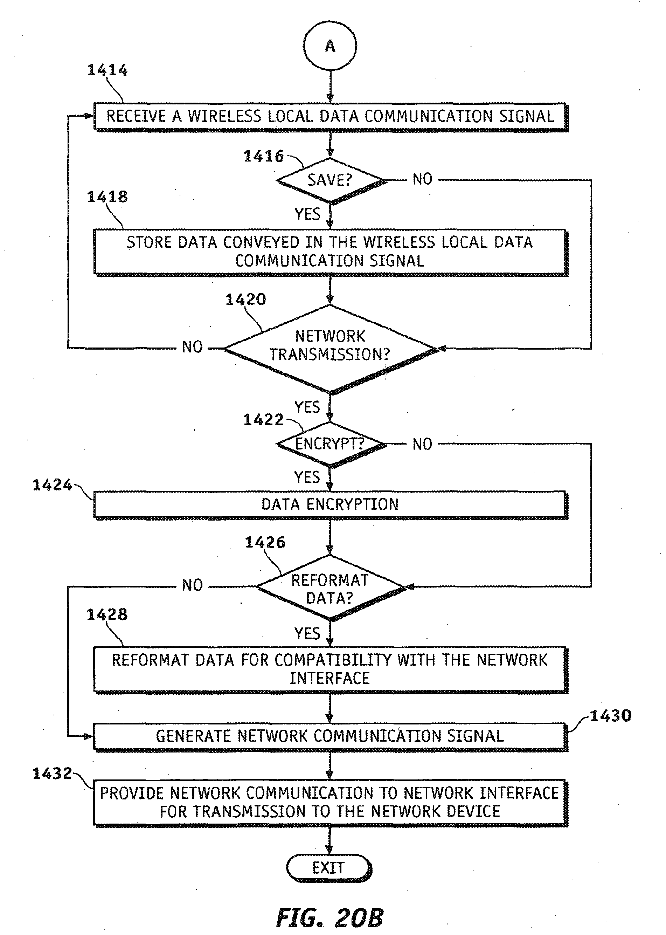

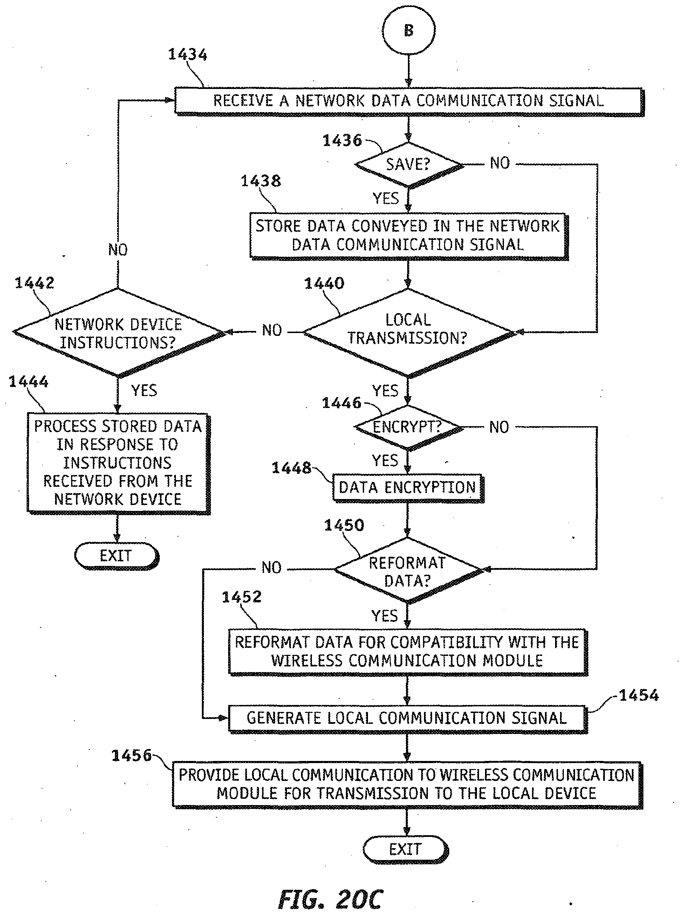

Translation device 1300 provides a wireless bridge between a local device and a network device, and translation device 1300 can support a range of data transmission and data storage features. In this regard, FIG. 20 is a flow chart that depicts an example data storage and translation process 1400 that may be supported by translation device 1300. The various tasks performed in connection with process 1400 may be performed by software, hardware, firmware, or any combination. For illustrative purposes, the following description of process 1400 may refer to elements mentioned above in connection with FIGS. 18 and 19. In practice, portions of process 1400 may be performed by different elements of the described system, e.g., wireless communication module 1308, memory element 1310, processing architecture 1312, or network interface 1316. It should be appreciated that process 1400 may include any number of additional or alternative tasks, the tasks shown in FIG. 20 need not be performed in the illustrated order, and process 1400 may be incorporated into a more comprehensive procedure or process having additional functionality not described in detail here.

Data storage and translation process 1400 may begin when the translation device is attached to a network device via the network interface of the translation device (task 1402). In this example, task 1402 is associated with the coupling of a USB-compatible translation device to a personal computer via the USB interface of the translation device. In response to this attachment, process 1400 powers the translation device and initializes the wireless communication module (task 1404). In accordance with conventional methodologies, the USB interface provides operating power from the computer to the translation device, and such operating power may be utilized to energize the wireless communication module and other functional elements of the translation device. In this example, the computer detects the mounting of the translation device and responds by automatically launching its host application (task 1406). Alternatively, the computer may prompt the user to manually launch the host application.

The translation device may be configured to support an auto-detect or standby mode, during which the translation device "listens" for compatible local devices that come within wireless transmission range. Such an auto device detection mode may be desirable to enable the system to accommodate intermittent or unreliable links by delaying wireless transmission of data until a link of sufficient strength is established. Such an auto device detection mode may also be desirable in a caregiver office environment to enable the system to download data (automatically or upon patient approval) whenever a patient enters the waiting room. If the auto device detection mode is active (query task 1408), then the translation device may check to determine whether a local device has been detected (query task 1410). If the translation device detects a local device within range, then data storage and translation process 1400 may continue as described below. Otherwise, the translation device may idle until it detects a local device within range, or process 1400 may be re-entered at query task 1408. If the auto device detection mode is inactive, or if the translation device does not support the auto device detection mode, then query task 1408 may lead to a query task 1412.

Data storage and translation process 1400 may perform query task 1412 to determine whether a user of the host application has assumed control over the translation device. If host control is not initiated, then process 1400 may be re-entered at query task 1408. Alternatively, if host control is not initiated, then process 1400 may idle until host control occurs. If, however, host control is initiated, then process 1400 may continue as described below.

Depending upon the implementation and the application, the translation device may receive and process data from a wireless local device and/or receive and process data from a network device. For ease of description, data storage and translation process 1400 is arbitrarily and artificially separated into sub-process A (relating to the handling of incoming wireless communication signals) and sub-process B (relating to the handling of incoming network communication signals). An embodiment of the translation device may be suitably configured to carry out both sub-processes concurrently or in a synchronous manner that avoids transmit/receive clashes. Either or both of these sub-processes may follow query task 1410 or query task 1412, as indicated in FIG. 20A.

Referring to sub-process A (see FIG. 20B), the translation device may receive a wireless local data communication signal from a local device within the infusion system (task 1414). In one example embodiment, during an initial handshaking or packet exchange routine, the device initiating contact indicates whether the transmission is a one-time packet (which could be sent as often as required) or a synchronous-link packet that requires time synchronization of packets sent and received between the two communicating devices. If data conveyed in the received wireless local data communication signal is to be saved (query task 1416), then the translation device may extract and store the data in its resident memory element (task 1418). Following the data storage of task 1418, data storage and translation process 1400 may proceed to a query task 1420. If data conveyed in the wireless local data communication signal is not to be saved, then process 1400 may bypass task 1418 and proceed to query task 1420.

Query task 1420 may determine whether the translation device is to perform network transmission of data. The translation device may be suitably configured to support network transmission of data stored in the memory element and/or network transmission of data that need not be stored in the memory element. For example, the translation device may be configured to process data stored in the memory element for transmission to a network device that is external to the infusion system. In this example, such network transmission corresponds to transmission of data from the translation device to the host computer via the USB interface. If network transmission has not been initiated, then data storage and translation process 1400 may be re-entered at task 1414 to allow the translation device to continue receiving wireless communication signals. If, however, network transmission has been initiated, then process 1400 may proceed to a query task 1422.

Query task 1422 determines whether the translation device is to perform data encryption. The translation device may be suitably configured to encrypt data conveyed in wireless local data communication signals, to encrypt data conveyed in network communication signals, and/or to encrypt data stored in the memory element. For example, the translation device may encrypt data stored in the memory element for encrypted transmission to the network device, which is compatibly configured to decrypt the data. If encryption is to be performed, then data storage and translation process 1400 performs data encryption (task 1424) using any suitable data encryption technique. After process 1400 performs encryption, it may lead to a query task 1426. If the data will not be encrypted, then process 1400 may bypass task 1424 and proceed to query task 1426.

Query task 1426 determines whether the translation device is to reformat data for transmission to the network device. For example, data storage and translation process 1400 may reformat data conveyed in the wireless local data communication signal for compatibility with the network interface (task 1428). Process 1400 may additionally (or alternatively) reformat data that has been stored in the memory element. Such reformatting may be desirable to enable the network interface to provide network communications to the network device, where the network communications convey the reformatted data. After reformatting data in a desired manner, the translation device can generate a network communication signal (task 1430). Task 1430 may also be performed if query task 1426 determines that reformatting is unnecessary or undesired. In this example, the network communication signal includes data that was conveyed in the wireless local data communication signal and/or data retrieved from the memory element.

Eventually, data storage and translation process 1400 provides the network communication signal (generated during task 1430) to the network interface for transmission to the network device (task 1432). In the example embodiment, task 1432 results in the transmission of data to the host computer via the USB interface. Following task 1432, process 1400 may exit or it may be re-entered at a designated point, such as query task 1408.

Referring to sub-process B (see FIG. 20C), the translation device may receive a network data communication signal from a network device that is external to the infusion system (task 1434). In one example embodiment, during an initial handshaking or packet exchange routine, the device initiating contact indicates whether the transmission is a one-time packet (which could be sent as often as required) or a synchronous-link packet that requires time synchronization of packets sent and received between the two communicating devices. If data conveyed in the network data communication signal is to be saved (query task 1436), then the translation device may extract and store the data in its resident memory element (task 1438). Thereafter, data storage and translation process 1400 may proceed to a query task 1440. If data conveyed in the network data communication signal is not to be saved, then process 1400 may bypass task 1438 and proceed to query task 1440.

Query task 1440 may determine whether the translation device is to perform local transmission of data. The translation device may be suitably configured to support local transmission of data stored in the memory element and/or local transmission of data that need not be stored in the memory element. For example, the translation device may be configured to process data stored in the memory element for transmission to a local device within the infusion system. In this example, such local transmission corresponds to transmission of data from the translation device to a local device via the wireless communication module. If local transmission has not been initiated, then data storage and translation process 1400 may check whether the received network data communication signal conveys operating or control instructions from the network device (query task 1442). If so, then the translation device may process data stored in the memory element in response to such instructions (task 1444). These instructions may include or indicate a request for certain data stored at the translation device, a request for the translation device to obtain data from a local device, programming or configuration data for the translation device and/or a local device, or the like. Following task 1444, process 1400 may exit or it may be re-entered at a designated point, such as task 1434 or query task 1408.

If query task 1440 determines that local transmission has been initiated, then data storage and translation process 1400 may proceed to a query task 1446. Query task 1446 determines whether the translation device is to perform data encryption as described previously. For example, the translation device may encrypt data conveyed in the received network data communication signal and/or data stored in the memory element for encrypted transmission to the wireless local device, which is compatibly configured to decrypt the data. If encryption is to be performed, then process 1400 performs data encryption (task 1448) using any suitable data encryption technique. After process 1400 encrypts the data, it may proceed to a query task 1450. If the data will not be encrypted, then process 1400 may bypass task 1448 and proceed to query task 1450.

Query task 1450 determines whether the translation device is to reformat data for transmission to the wireless local device. For example, data storage and translation process 1400 may reformat data conveyed in the network data communication signal for compatibility with the wireless data communication module (task 1452). Process 1400 may additionally (or alternatively) reformat data that has been stored in the memory element. Such reformatting may be desirable to enable the wireless communication module to provide local wireless communication signals to the local device(s), where the wireless signals convey the reformatted data. After reformatting data in a desired manner, the translation device can generate a local communication signal (task 1454). Task 1454 may also be performed if query task 1450 determines that reformatting is unnecessary or undesired. In this example, the local communication signal is a wireless signal that includes data that was conveyed in the network data communication signal and/or data retrieved from the memory element.

Eventually, data storage and translation process 1400 provides the local communication signal (generated during task 1454) to the wireless communication module for transmission to the local device (task 1456). In the example embodiment, task 1456 results in the wireless transmission of data to a local device via the wireless communication module. Following task 1456, process 1400 may exit or it may be re-entered at a designated point, such as query task 1408.

Translation device 1300, data storage and translation process 1400, and other processes supported by translation device 1300 provide added flexibility and convenience for users of the infusion system. For example, translation device 1300 can support the downloading of history data from an infusion pump or an infusion pump monitor with automatic storage to its internal flash memory. Such downloading may be driven by the host application - the host computer can command translation device 1300 to download data to the flash memory - for retrieval and analysis at a later date by the patient's caregiver. Patient history data may be encrypted such that only an authorized caregiver computer system can access the history files. Alternatively, the history files could be read-only by the patient, with read/write access provided to the caregiver. In example embodiments, the host application may be configured to detect whether the patient or a caregiver is communicating with the local device via translation device 1300. Consequently, translation device 1300 may be configured to support patient-specific and/or caregiver-specific functions and operations if so desired.

Depending upon the given deployment of an infusion system, it may be desirable to collect data from a plurality of local devices such that the collected data can be stored, processed, routed, or otherwise managed in an controlled manner. In this regard, FIG. 21 is a schematic representation of an example network deployment of a wireless telemetry router 1500 configured in accordance with an example embodiment of the invention. Wireless telemetry router 1500 may be deployed in a medical device system such as network-based infusion system 100 (see FIG. 1). Wireless telemetry router 1500 is suitably configured to communicate with a plurality of wireless devices within a local medical device system, such as a local infusion system. Wireless telemetry router 1500 is also configured to communicate with one or more network devices, which may be external to the local medical device system. For example, wireless telemetry router 1500 may communicate with network devices coupled to wireless telemetry router 1500 via an Ethernet connection and/or via wireless links.

The flexible nature of the example environment is depicted in FIG. 21, which depicts wireless telemetry router 1500 in communication with a variety of devices. In an example embodiment, wireless telemetry router 1500 may be suitably configured to communicate with one or more of the following devices, without limitation: a plurality of physiological characteristic sensor transmitters 1502, a wireless personal digital assistant 1504, a wireless laptop computer 1506, a network monitor 1508, a network computer 1510, a network personal digital assistant 1512, a network hospital management system 1514, and a network printer 1516. Wireless telemetry router 1500 may also be configured to support communication with the various local devices and network devices mentioned in the above description of infusion system 100.

Although FIG. 21 depicts five physiological characteristic sensor transmitters 1502, wireless telemetry router 1500 can support any number of sensor transmitters (limited only by practical operating restrictions such as bandwidth, available power, transmission range, etc.). Each physiological characteristic sensor transmitter 1502 is suitably configured to measure a physiologic characteristic of a patient. In the example infusion system described here, each sensor transmitter 1502 is a continuous glucose (e.g., blood glucose) sensor transmitter that measures the glucose level of a patient in real time. Each sensor transmitter 1502 may be realized in a form that is intended to be worn by the patient, attached to the patient's skin, implanted within the patient's body, or the like. Each sensor transmitter 1502 includes a wireless transmitter that facilitates transmission of physiologic sensor data of the user to wireless telemetry router 1500 and possibly other devices within the local infusion system.

Wireless telemetry router 1500 may be deployed in any environment where physiological characteristic sensor transmitters 1502 might come in range. Wireless telemetry router 1500 can support a system where a plurality of sensor transmitters 1502 are used by one person and/or a system that contemplates more than one person (each using only one sensor transmitter 1502). Moreover, wireless telemetry router 1500 can be suitably configured to support different types of sensor transmitters, and the example environment depicted in FIG. 21 need not be limited to an insulin infusion system or any specific type of medical device system. Example applications of wireless telemetry router 1500 include the following, without limitation: one patient having multiple sensor transmitters 1502, each being configured to provide data indicative of a different physiologic characteristic; a home deployment where more than one member of a family uses a sensor transmitter 1502; a school deployment where it may be desirable to monitor the physiologic data for any number of students; a hospital deployment where it may be desirable to monitor physiologic data for any number of patients; or a caregiver office environment where it may be desirable to identify specific sensor transmitters 1502 for purposes of patient identification and/or to obtain data from sensor transmitters 1502.

Physiological characteristic sensor transmitters 1502 and wireless telemetry router 1500 are suitably configured to support wireless data communication via respective wireless links 1518, which may be unidirectional (as shown) or bidirectional, depending upon the particular system and/or the specific type of sensor transmitters 1502. Accordingly, wireless telemetry router 1500 includes a suitably configured wireless communication module that is capable of supporting multiple sensor transmitters 1502.

Although not a requirement of the system, wireless links 1518 may be established using the same wireless data communication protocol and wireless data transmission scheme. Wireless telemetry router 1500 may utilize any number of suitable wireless data communication protocols, techniques, or methodologies for wireless links 1518, including, without limitation: RF; IrDA (infrared); Bluetooth; ZigBee (and other variants of the IEEE 802.15 protocol); IEEE 802.11 (any variation); IEEE 802.16 (WiMAX or any other variation); Direct Sequence Spread Spectrum; Frequency Hopping Spread Spectrum; cellular/wireless/cordless telecommunication protocols; wireless home network communication protocols; paging network protocols; magnetic induction; satellite data communication protocols; wireless hospital or health care facility network protocols such as those operating in the WMTS bands; GPRS; and proprietary wireless data communication protocols such as variants of Wireless USB. In the example embodiment, wireless links 1518 are carried over the 900-930 MHz band that is reserved for industrial, scientific, and medical equipment use. As another example, wireless links 1518 in a hospital implementation may utilize the WMTS bands that are reserved for hospital applications. Packaging of sensor data, error detection, security, sensor transmitter identification, and other sensor data processing techniques may be governed by known or proprietary protocols.

Wireless telemetry router 1500 may be configured to communicate with network devices via Ethernet connectivity (or via any suitable data communication methodology). FIG. 21 depicts an Ethernet data communication architecture 1520 that links wireless telemetry router 1500 to network monitor 1508, network computer 1510, network personal digital assistant 1512, network hospital management system 1514, and network printer 1516. Of course, these example network devices are not exhaustive, and embodiments of the invention are not limited to these examples. A given link between wireless telemetry router 1500 and a network device may be unidirectional (in either direction) or bidirectional, depending upon the particular system and/or the specific type of network device. For example, the link from wireless telemetry router 1500 to network printer 1516 may be unidirectional, the link from wireless telemetry router 1500 to network monitor 1508 may be unidirectional, and other links may be bidirectional.

Wireless telemetry router 1500 may be configured to support wireless communication with compatible wireless devices, such as wireless personal digital assistant 1504 and wireless laptop computer 1506. Accordingly, wireless telemetry router 1500 includes a suitably configured wireless communication module, which may (but need not) be distinct from the wireless communication module that receives wireless links 1518. In this regard, FIG. 21 depicts wireless links 1522 between wireless telemetry router 1500 and these wireless devices. A given wireless link 1522 between wireless telemetry router and a wireless device may be unidirectional in either direction or bidirectional (as shown in FIG. 21), depending upon the particular system and/or the specific type of wireless device. In practice, wireless links 1522 enable wireless telemetry router 1500 to communicate directly with wireless devices while bypassing the network (i.e., without having to traverse Ethernet data communication architecture 1520).

Although not a requirement of the system, wireless links 1522 may be established using the same wireless data communication protocol and wireless data transmission scheme. In this example, wireless telemetry router 1500 utilizes one wireless data communication technique for wireless links 1522 and a different wireless data communication technique for wireless links 1518. Wireless telemetry router 1500 may utilize any number of suitable wireless data communication protocols, techniques, or methodologies for wireless links 1522, including, without limitation: RF; IrDA (infrared); Bluetooth; ZigBee (and other variants of the IEEE 802.15 protocol); IEEE 802.11 (any variation); IEEE 802.16 (WiMAX or any other variation); Direct Sequence Spread Spectrum; Frequency Hopping Spread Spectrum; cellular/wireless/cordless telecommunication protocols; wireless home network communication protocols; paging network protocols; magnetic induction; satellite data communication protocols; wireless hospital or health care facility network protocols such as those operating in the WMTS bands; GPRS; and proprietary wireless data communication protocols such as variants of Wireless USB. Packaging of data, error detection, security, and other data processing techniques may be governed by known or proprietary protocols.

In one example embodiment, wireless telemetry router 1500 includes an HTML-based setup, management, and control interface that can be accessed via any authorized computer or device having HTML browser capabilities and connectivity to wireless telemetry router 1500. For example, an administrator may be able to access wireless telemetry router 1500 via the Internet and a conventional web browser application residing on wireless personal digital assistant 1504, wireless laptop computer 1506, network computer 1510, or network personal digital assistant 1512. The control interface may be provided as one or more HTML pages that reside in the firmware/software of wireless telemetry router 1500. The control interface can be accessed using an IP address and/or a network interface card that is unique to that particular wireless telemetry router 1500. Password and firewall protection may be implemented to provide protection against external misuse or data theft.

In connection with a setup procedure, wireless telemetry router 1500 may be provided with sensor identifiers for the respective physiological characteristic sensor transmitters 1502. The sensor identifiers may be, for example, the serial numbers of sensor transmitters 1502 or any information that uniquely distinguishes the different sensor transmitters 1502 within the operating environment. In example embodiments, wireless communication signals generated by an originating sensor transmitter 1502 conveys the corresponding sensor identifier. Wireless telemetry router 1500 can then process the sensor identifiers in a suitable manner. For example, wireless telemetry router 1500 may receive a wireless communication signal from an originating sensor transmitter 1502, obtain or extract the sensor identifier for that wireless communication signal, and process the sensor data conveyed in that wireless communication signal in a manner that is determined, governed, or dictated by the particular sensor identifier. This technique enables wireless telemetry router 1500 to identify the originating sensor transmitter 1502, the originating patient, the sensor transmitter type, or other pertinent information. Wireless telemetry router 1500 may then process, store, and/or route the sensor data in an appropriate manner. As another example, wireless telemetry router 1500 may receive a first wireless communication signal from a first sensor transmitter 1502a, receive a second wireless communication signal from a second sensor transmitter 1502b, obtain or extract the two respective sensor identifiers (which should be different), and process the sensor data conveyed in the two wireless communication signals in a synchronized manner that is determined, governed, or dictated by the sensor identifiers. This technique enables wireless telemetry router 1500 to prioritize the receipt, processing, storage, and/or transmission of sensor data depending upon the originating source.

In connection with a setup procedure, wireless telemetry router 1500 may be provided with network identifiers (e.g., IP addresses or network interface card identifiers) for the various destination network devices. Such network identifiers enable wireless telemetry router 1500 to determine how to process, handle, store, or route the received sensor data. In this regard, wireless telemetry router 1500 may, for example, maintain or access a lookup table (or any suitable memory or database structure) that contains the different sensor identifiers and a corresponding list of destination network identifiers for each sensor identifier. This lookup table may also include corresponding processing instructions for each sensor identifier.

Wireless telemetry router 1500 is generally configured to receive sensor data and route the sensor data to one or more destination network devices. In this example, wireless telemetry router 1500 receives a plurality of wireless communication signals from a plurality of physiological characteristic sensor transmitters 1502, where each wireless communication signal conveys sensor data generated by a respective sensor transmitter 1502. As mentioned above, each wireless communication signal may also convey a sensor identifier that uniquely identifies the originating sensor transmitter 1502. Wireless telemetry router 1500 can then process the received information in an appropriate manner, depending upon the particular application and the identity of the originating sensor transmitter 1502.

Wireless telemetry router 1500 may perform one or more operations on the received sensor data, including, without limitation: storing at least some of the sensor data (at wireless telemetry router 1500 itself or at a network device that is coupled to wireless telemetry router 1500); forward at least some of the sensor data to a destination network device; reformat data conveyed in the wireless communication signals for compatibility with a designated network data communication protocol; or process at least some of the sensor data. In example embodiments, wireless telemetry router 1500 may include some functionality and processing intelligence that might normally be found elsewhere in the system environment. For example, wireless telemetry router 1500 may be configured to receive uncalibrated physiologic characteristic data, such as an uncalibrated patient glucose level, and calibrate the data before routing it to the destination network device.

In connection with its routing function, wireless telemetry router 1500 may generate a network communication that complies with a specified network data communication protocol. The network communication conveys sensor data, which may include stored sensor data, real-time sensor data that is being immediately routed, or a combination thereof. Wireless telemetry router 1500 can then transmit the network communication to one or more network devices. Wireless telemetry router 1500 transmits the network communication in accordance with the selected network data communication protocol and in accordance with the selected data transmission technique. For example, wireless telemetry router 1500 may function as a translation device between data received on wireless links 1518 (using one protocol and transmission scheme combination) and data transmitted over Ethernet data communication architecture 1520 (using another protocol and transmission scheme combination). As another example, wireless telemetry router 1500 may function as a translation device between data received on wireless links 1518 (using one protocol and transmission scheme combination) and data transmitted over wireless links 1522 (using another protocol and transmission scheme combination).

Wireless telemetry router 1500 may also be configured to generate warning, error, alarm, and alert information ("diagnostic information"), which may be routed using the techniques described above. The diagnostic information may be displayed or rendered at wireless telemetry router 1500 itself and/or routed for display or rendering at a network device. The diagnostic information may include, without limitation: information related to the operation or status of wireless telemetry router 1500; information related to the operation or status of physiological characteristic sensor transmitters 1502; information related to the operation or status of a network device; or any of the notifications, alerts, alarms, or status reports described in more detail above.

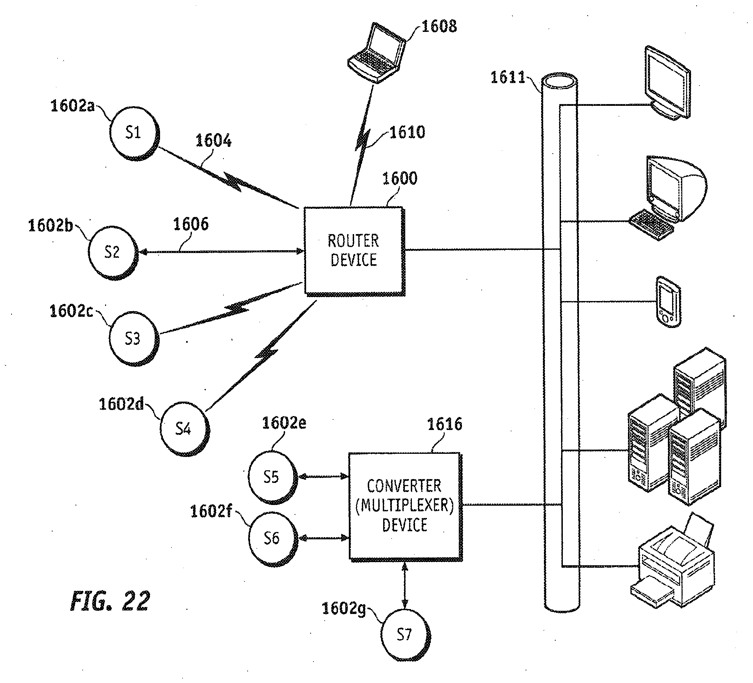

FIG. 22 is a schematic representation of a deployment of a network router device 1600 configured in accordance with an alternate embodiment. Certain features, functions, and elements of router device 1600 may be as described above for wireless telemetry router 1500; such common features, functions, and elements will not be redundantly described here in the context of router device 1600.

Network router device 1600 provides centralized processing, management, and routing of data obtained from medical devices. Although router device 1600 can support different types of medical devices, the following description assumes (for simplicity) that the medical devices are all of the same type, namely, physiological sensor transceivers 1602. Router device 1600 can communicate with sensor transceivers 1602 in different ways to suit the needs of the particular deployment and/or to suit the particular system implementation and configuration. Although FIG. 22 depicts seven sensor transceivers 1602, router device 1600 can support any number of physiological characteristic sensor transceivers in a wireless communication mode and/or a wired communication mode (limited only by practical operating restrictions such as bandwidth, available power, transmission range, etc.). Moreover, any given sensor transceiver 1602 may be capable of unidirectional data communication or bidirectional data communication with network router device 1600 and/or with one or more network appliances (described below) coupled to router device 1600. Accordingly, each sensor transceiver 1602 may include a wireless transceiver and a suitably configured wired data communication interface.

In the following example, each physiological characteristic sensor transceiver 1602 is a glucose sensor transceiver as described in more detail above, and the sensor measurement values generated by the glucose sensor transceivers represent the raw glucose sensor data. For a given glucose sensor value, network router device 1600 generates a calibrated physiological characteristic measurement value from a patient calibration value and the glucose sensor value. In the following example, the patient calibration values are glucose meter measurement values, and the calibrated physiological characteristic measurement values are blood glucose levels. This glucose sensor monitoring example is not intended to limit or restrict the scope of the embodiments described and claimed herein.

FIG. 22 depicts a number of different data communication possibilities for ease of description. For example, sensor transceiver 1602a communicates with network router device 1600 over a wireless data communication link 1604, while sensor transceiver 1602b communicates with router device 1600 over a wired data communication link 1606. Wireless data communication link 1604 (and other wireless links employed by router device 1600) may be established and maintained as described above for wireless links 1518. In preferred embodiments for deployment in the United States, wireless data communication links between sensor transceivers 1602 and router device 1600 employ carrier frequencies between 900 MHz and 930 MHz, such as 916.5 MHz. In Europe, a carrier frequency of 868.35 MHz is used. In certain embodiments, WMTS frequency bands can be utilized (e.g., 608-614 MHz, 1395-1400 MHz, or 1429-1432 MHz). Wired data communication link 1606 (and other wired links employed by router device 1600) may be configured as described above in the context of monitor 500.

Network router device 1600 may also be configured to communicate with one or more mobile network devices (e.g., a mobile network device 1608) via wireless data communication links (such as wireless link 1610). These wireless data communication links may be established and maintained as described above for wireless links 1518. In preferred embodiments, wireless data communication links between router device 1600 and mobile network devices are IEEE 802.11 compliant links. Router device 1600 can also communicate with one or more network devices via a traditional wired Ethernet data communication link, over any suitable network topology, via the Internet, or the like. In general, network communications may be handled by a suitably configured network architecture 1611, which represents any number of interconnecting links, network elements, switching components, or the like.

Network router device 1600 may be configured to communicate with one or more network appliances that are coupled to router device 1600 via network architecture 1611. One such network appliance is a converter/multiplexer device 1616 that can receive data from multiple sensor transceivers and process that data for serial transmission to network router device 1600 via network architecture 1611. For this example, FIG. 22 depicts converter/multiplexer device 1616 coupled to three sensor transceivers (1602e, 1602f, and 1602g). Converter/multiplexer device 1616 can be realized as a component having connection ports and interfaces for receiving data packets via cables from the sensor transceivers. The data packets may be transmitted in a serial fashion using any suitable protocol. Converter/multiplexer device 1616 may also be configured to handle data communications from network architecture 1611 to sensor transceivers 1602e, 1602f, or 1602g. In this embodiment, converter/multiplexer device 1616 utilizes wired links for data communication with its respective sensor transceivers 1602.

Although FIG. 22 includes only one network router device 1600, a system embodiment may utilize a plurality of such network router devices to provide "roaming" coverage throughout an area. In other words, the network environment can include multiple router devices coupled to network architecture 1611 in a manner that still allows centralized processing, management, and formatting of the received data as described in more detail below. The network environment can leverage suitable techniques and technologies to achieve the various data communication settings for each router device and to enable the multiple router devices to interact and cooperate with one another.

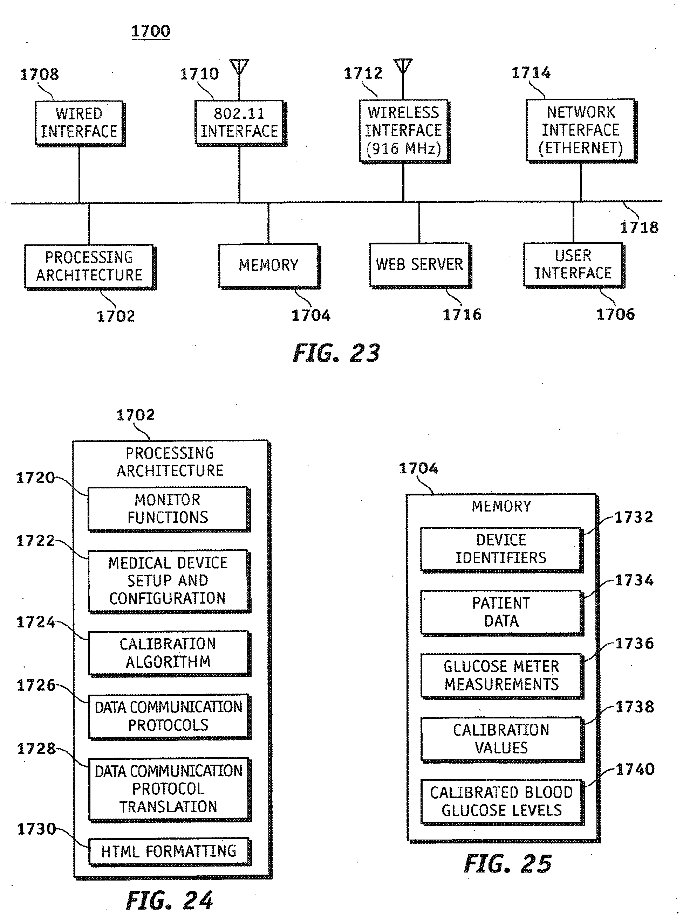

FIG. 23 is a schematic representation of a network router device 1700 suitable for use in a networked deployment as described above. For example, router device 1700 may be utilized in the network environment shown in FIG. 22. In this example, router device 1700 includes, without limitation: a processing architecture 1702; a suitable amount of memory 1704; a user interface 1706; a wired local data communication interface 1708; a wireless network data communication interface 1710; a wireless local data communication interface 1712; a wired network data communication interface 1714; and a web server 1716. These elements of router device 1700 may be coupled together via a bus 1718 or any suitable interconnection architecture. In practice, router device 1700 will include a number of additional components and features that perform known functions and/or that are unrelated to the operations described here.

Processing architecture 1702 may be generally configured and implemented as described above for processing architecture 514 (see FIG. 5). However, processing architecture 1702 includes processing logic that is suitably configured to carry out the functions, features, and operations associated with the network router device described herein. In this regard, FIG. 24 depicts a number of processing modules, functions, and operations that may be managed or carried out by processing architecture 1702. For this example embodiment, processing architecture 1702 includes processing logic related to: monitor functions 1720; medical device setup and configuration 1722; sensor data calibration algorithm(s) 1724; data communication protocols 1726; data communication protocol translation 1728; and HTML document formatting 1730. In practice, network router device 1700 need not employ all of these features and functions; FIG. 24 depicts a full-featured embodiment for ease of description.

Monitor functions 1720 enable processing architecture 1702 to carry out operations that are traditionally performed by a BG monitor system (such as sensor data calibration, reporting, alarm generation, graphing/charting, etc.) for a plurality of patients. In practice, monitor functions 1720 may include any of the functions described above in the context of the different monitor embodiments.

Processing architecture 1702 may be configured to perform medical device setup and configuration 1722 to initialize, setup, adjust, and/or configure sensor transceivers for operation with network router device 1700. In preferred embodiments, medical device setup and configuration functions 1722 can be performed remotely via web server 1716.

Processing architecture 1702 can execute one or more appropriate sensor data calibration algorithms 1724 that converts raw glucose sensor values into usable BG levels that have meaning to the patients or caregivers. As described in more detail here, calibration algorithms 1724 may process glucose meter measurement values for the different patients. Moreover, network router device 1700 may utilize a different calibration algorithm 1724 for each patient.

Data communication protocols 1726 enable network router device 1700 to transmit and receive data using different transport mechanisms and formats. For example, data communication protocols 1726 may be utilized to enable data communication in compliance with: a proprietary wireless scheme; USB technology; IEEE 802.11; an Ethernet scheme; or any of the wired or wireless data communication protocols mentioned here.

Processing architecture 1702 may also be configured to perform data communication protocol translation 1728 as needed. Such translation allows network router device 1700 to receive incoming data in accordance with a first data communication protocol, "repackage" the data in accordance with a second data communication protocol, and transmit the data as an outgoing communication using the second data communication protocol. Protocol translations may be performed in the uplink and/or the downlink direction.

As described in more detail below, processing architecture 1702 can perform data routing to any number of network computing devices. In some embodiments, processing architecture 1702 manages the serving of web pages to remote browser applications, where such web pages contain BG levels, sensor data, and/or patient data for one or more patients. HTML formatting 1730 can be utilized in this context to generate HTML documents (web pages) that contain the desired information.

Memory 1704 may be generally configured and implemented as described above for memory 516 (see FIG. 5). In this regard, memory 1704 can be coupled to processing architecture 1702 such that processing architecture 1702 can read information from, and write information to, memory 1704. In the alternative, memory 1704 may be integral to processing architecture 1702. As an example, processing architecture 1702 and memory 1704 may reside in an ASIC. In this embodiment, memory 1704 may be utilized as a centralized repository for calibrated BG levels, sensor data, and/or patient data corresponding to any number of local devices (within practical limitations). In this regard, FIG. 25 depicts different types of data that may be stored in memory 1704. Memory 1704 may be suitably configured to store, without limitation: device identifiers 1732 that identify different medical devices; patient data 1734; blood glucose measurements 1736; sensor calibration values or information 1738; and calibrated BG levels 1740. In practice, network router device 1700 need not store all of these data types; FIG. 25 depicts a full-featured embodiment for ease of description. These data elements are described in more detail below.

User interface 1706 may include one or more features that enable direct user interaction with network router device 1700. For example, user interface 1706 may include a keypad, keys, buttons, switches, lights, a display element, knobs, a touchpad, a joystick, a pointing device, a virtual writing tablet, or any device, component, or function that enables a user to select options, input information, or otherwise control the operation of router device 1700.

Wired local data communication interface 1708 represents hardware, software, firmware, and/or processing logic that is configured to receive (and transmit) communication signals over a wired link from (and to) medical devices such as physiological characteristic sensor transceivers. Interface 1708 may include a plurality of ports that facilitate concurrent data transfer with a plurality of sensor transceivers. In practice, interface 1708 can be configured to support one or more of the wired data communication schemes described above in the context of monitor 500 (see FIG. 5). In one embodiment, interface 1708 supports a serial data packet transmission scheme.

Wireless local data communication interface 1712 represents hardware, software, firmware, and/or processing logic that is configured to receive (and transmit) wireless communication signals over a wireless link from (and to) medical devices such as physiological characteristic sensor transceivers. Interface 1712 is preferably configured to support data transfer with a plurality of sensor transceivers (interface 1712 is compliant with the particular wireless data communication protocol(s) used by the sensor transceivers). In practice, interface 1712 can be configured to support one or more of the wireless data communication protocols described above in the context of wireless links 1518. In one embodiment, interface 1712 transmits data using a carrier frequency in the 900-930 MHz band; in the United States, the carrier frequency is 916 MHz.

In the uplink direction (i.e., from the sensor transceiver to network router device 1700), the communication signals received by wired local data communication interface 1708 and/or wireless local data communication interface 1712 convey raw glucose sensor data. Uplink communication signals may additionally (or alternatively) convey other patient data, device status data, or other information to be utilized by the networked system. In the downlink direction (i.e., from router device 1700 to a sensor transceiver), the communication signals transmitted by interface 1708 and/or interface 1712 may convey query messages that prompt the transmission of sensor data, device configuration messages that contain configuration data for the medical devices, device initialization messages that contain initialization data for the medical devices, upgrade software for sensor transceivers, or the like.

Wireless network data communication interface 1710 represents hardware, software, firmware, and/or processing logic that is configured to transmit (and receive) wireless communication signals over a wireless link to (and from) network devices such as a remote computing device. Interface 1710 employs an appropriate network data communication protocol to exchange network communications with the network architecture coupled to the network devices. In practice, interface 1710 can be configured to support one or more of the wireless data communication protocols described above in the context of wireless links 1518. In one embodiment, interface 1710 is compliant with an IEEE 802.11 protocol.

Wired network data communication interface 1714 represents hardware, software, firmware, and/or processing logic that is configured to transmit (and receive) data communication signals over a wired link to (and from) network devices such as a remote computing device. Interface 1714 employs an appropriate network data communication protocol to exchange network communications with the network architecture coupled to the network devices. In practice, interface 1714 can be configured to support one or more of the wired data communication schemes described above in the context of monitor 500. In one embodiment, interface 1714 is compliant with an Ethernet protocol.

In the uplink direction (i.e., from network router device 1700 to the network architecture), the network communications transmitted by wireless network data communication interface 1710 and/or wired network data communication interface 1714 can convey the calibrated BG levels corresponding to different raw glucose sensor values. Such uplink communications may additionally (or alternatively) convey other patient data, device status data, alerts, packet acknowledgements, or other information to be utilized by the networked system. In preferred embodiments, uplink communications from router device 1700 may be formatted as HTML documents (web pages) that contain the desired information. In the downlink direction (i.e., from the network architecture to router device 1700), the network communications received by interface 1710 and/or interface 1714 may convey requests, messages, or data associated with the management, control, or configuration of router device 1700 or the sensor transceivers that communicate with router device 1700. For example, downlink communication signals may convey, without limitation: requests to store patient or sensor data; requests for HTML documents (URL requests); patient calibration values such as glucose meter measurements; medical device configuration requests; medical device initialization requests; setup requests for router device 1700; ; upgrade software for sensor transceivers; upgrade software for router device 1700; or the like.

Web server 1716 represents hardware, software, firmware, and/or processing logic that is configured to provide HTML documents (web pages) to network computing devices. Network router device 1700 is preferably configured to transmit HTML documents to web browser applications running on the network computing devices. As mentioned above in connection with wireless telemetry router 1500, web server 1716 may also serve as an HTML-based setup, management, and control interface that can be accessed via any authorized computer or device having HTML browser capabilities and connectivity to router device 1700. In preferred embodiments web server 1716 leverages existing web server technology; the details of such technology will not be described here.

Network router device 1700 can provide centralized data storage for one or more patients. In one embodiment, router device 1700 utilizes its internal memory 1704 or a locally attached memory storage device for such centralized data storage. Alternatively (or additionally), router device 1700 may be coupled to a suitably configured network storage device or subsystem that provides such centralized data storage. Regardless of the particular memory configuration, router device 1700 may be suitably configured to support a plurality of different medical devices for a plurality of different patients. In practice, each medical device is uniquely identified (within at least the network environment arid possibly on an absolute global scale) with a corresponding device identifier, which may be the serial number of the device, an arbitrarily assigned alphanumeric string, or the like. Data communication signals generated by a given medical device may contain the device identifier for that medical device, which allows router device 1700 to determine the source of the data communication signals. As depicted in FIG. 25, memory 1704 may store a list or a table of device identifiers 1732 and link the device identifiers 1732 to their respective data sets.

Each device identifier 1732 may be linked to other information stored in memory 1704. This allows network router device 1700 to maintain separate records for each medical device or for each patient. For example, each device identifier 1732 may be linked to respective patient data 1734, which may include, without limitation: the raw sensor data obtained from the medical device; the name of the patient; the hospital room number of the patient; the hospital bed number of the patient; the name of the patient's caregiver; phone numbers for the patient; or the like. In addition, each device identifier 1732 may be linked to data associated with the particular type of medical device and/or the particular type of medical device system. In this example, each device identifier 1732 may associated with one or more glucose meter measurements 1736 obtained from a calibrating device other than the glucose sensor itself; calibration values or quantities 1738 that are based on the glucose meter measurements 1736; and calibrated BG levels 1740 that represent usable information that can be provided to an end user. The calibrated BG levels 1740 are calculated from the raw glucose sensor data and the respective calibration values 1738.

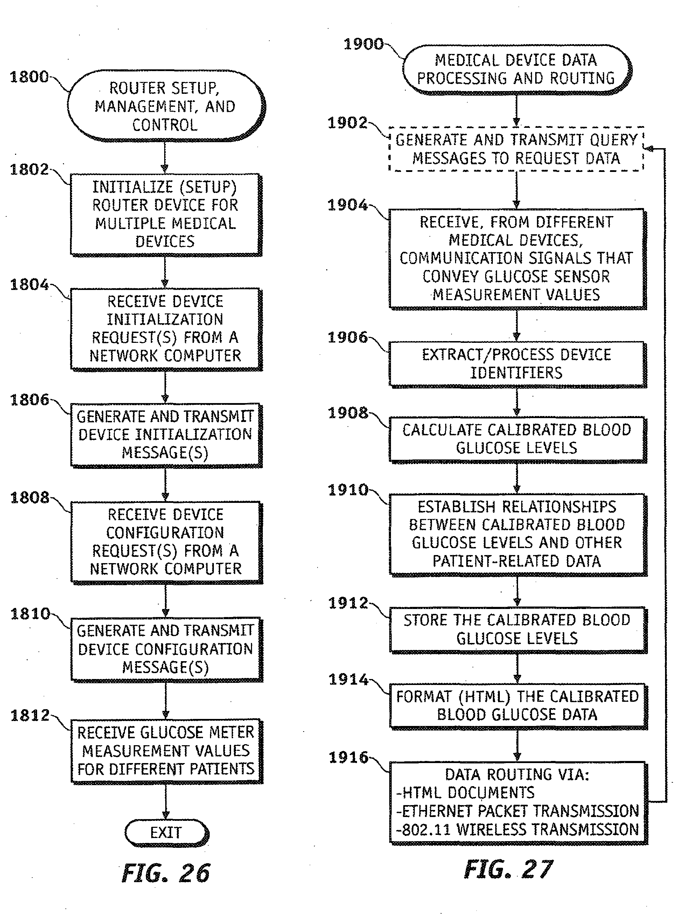

FIG. 26 is a flow chart of a setup, management, and control process 1800 suitable for use with a network router device as described here. Process 1800 represents one example technique for operating a network-based medical device system. A system may be able to support any number of alternative techniques and methodologies, and the following description of process 1800 is not intended to limit the scope or application of the invention in any way. The various tasks performed in connection with process 1800 may be performed by software, hardware, firmware, or any combination. For illustrative purposes, the following description of process 1800 may refer to elements mentioned above in connection with FIGS. 21-25. It should be appreciated that process 1800 may include any number of additional or alternative tasks, the tasks shown in FIG. 26 need not be performed in the illustrated order, and process 1800 may be incorporated into a more comprehensive procedure or process having additional functionality not described in detail here.

Setup, management, and control process 1800 may begin by initializing the router device for use with multiple medical devices (task 1802). Initializing the router device may be carried out by a suitable web-based setup procedure where the router device generates appropriate web pages for display at a remote computing device. Referring to FIG. 24, medical device setup and configuration functions 1722 of processing architecture 1702 may be utilized in connection with task 1802. During task 1802, a web server in the router device may generate one or more setup pages that facilitate setup configuration of the router device itself. For example, these setup pages may enable the system administrator to configure IP addresses, NIC card addresses, passwords, Ethernet and wireless interface settings, or the like. During task 1802, the router device may also generate one or more setup pages having data entry fields for establishing a new patient profile (e.g., fields for the patient's name, date of birth, patient identification number, room number, bed number, etc.).

Process 1800 may also be utilized to setup or initialize new medical devices for communication with the router device. In this regard, the router device can generate device setup pages that allow the patient or caregiver to add a new medical device to the system. In one embodiment, the serial number of the new medical device serves as a unique identifier within the network environment, and the serial number (which may be located on the medical device itself) is entered into an appropriate data entry field on a device setup page.

At this time, the device setup procedure may also prompt the user or caregiver for one or more device-specific settings. In this example, the router device generates a web page that prompts the user or caregiver to enter hyperglycemic and hypoglycemic alarm levels for the patient. Once all of the necessary information has been entered, the router device may receive a device initialization request (task 1804) from the web browser application at the respective network computer. Depending upon the system implementation and the current operating conditions, the device initialization request will be received via a wired network data communication interface (such as wired network data communication interface 1714) or a wireless network data communication interface (such as wireless network data communication interface 1710).

In response to the device initialization request, the router device may generate and transmit a suitably formatted device initialization message to the specified medical device (task 1806). This message may convey device initialization data that is processed by the medical device in connection with the device setup procedure. Depending upon the system implementation and the current operating conditions, the device initialization message will be transmitted to the medical device via a wired local data communication interface (such as wired local data communication interface 1708) or a wireless local data communication interface (such as wireless local data communication interface 1712). The medical device may respond to the device initialization message by confirming that the correct device identifier has been entered, by establishing a test communication link with the router device, or the like. Assuming that the device initialization is successful, the router device will be able to receive data for the new patient and for the new medical device.

Process 1800 may also allow a remote user or caregiver to adjust settings of a medical device via the router device. Referring to FIG. 24, medical device setup and configuration functions 1722 of processing architecture 1702 may be utilized in connection with the adjustment of these settings. For example, the router device may receive a configuration request (task 1808) that originates from a computing device coupled to the router device. In this embodiment, the configuration request is generated from the web browser application at a respective network computer (the router device can generate a suitably formatted diagnostic screen that enables interaction with device settings). Depending upon the system implementation and the current operating conditions, the device configuration request will be received via a wired network data communication interface (such as wired network data communication interface 1714) or a wireless network data communication interface (such as wireless network data communication interface 1710).

In response to the device configuration request, the router device may generate and transmit a suitably formatted device configuration message to the specified medical device (task 1810). This message may convey device configuration data for the medical device, where the configuration data influences the operation of the medical device. For a continuous glucose sensor transceiver device, the configuration data may include or be related to: patient identifying information; automatic power off settings; carrier frequency or channel selection settings; transmit power settings; settings related to the storing and batch transmission of historical device data; or the like. Depending upon the system implementation and the current operating conditions, the device initialization message will be transmitted to the medical device via a wired local data communication interface (such as wired local data communication interface 1708) or a wireless local data communication interface (such as wireless local data communication interface 1712). The medical device may respond to the device configuration message by changing its operating parameters, settings, or variables in an appropriate manner.

Process 1800 can also generate patient calibration values from calibrating measurements. Referring to FIG. 24, calibration algorithm(s) 1724 associated with processing architecture 1702 may be utilized in connection with this feature. For the glucose monitoring application described here, process 1800 independently receives glucose meter measurement values for one or more different patients (task 1812) and generates respective calibration values or factors from the glucose meter measurement values. The glucose meter measurement values (and/or the patient calibration values) may be received by the router device via a wired or a wireless data communication interface, either directly from a meter device or via the network architecture, for example, in response to a web-based data entry application maintained by network router device 1700. As mentioned above, the glucose meter measurement values may be, for example, fingerstick measurements taken directly from patient blood samples. The glucose meter measurements and/or the corresponding calibration values may be stored in memory for subsequent retrieval or processing by the router device. Process 1800 may exit or be re-entered at an appropriate point following task 1812.

Once the medical devices have been initialized, they can send data to the router device for storage, processing, formatting, and handling. FIG. 27 is a flow chart of a data processing and routing process 1900 suitable for use with a network router device as described herein. Process 1900 represents one example technique for operating a network-based medical device system. A system may be able to support any number of alternative techniques and methodologies, and the following description of process 1900 is not intended to limit the scope or application of the invention in any way. The various tasks performed in connection with process 1900 may be performed by software, hardware, firmware, or any combination. For illustrative purposes, the following description of process 1900 may refer to elements mentioned above in connection with FIGS. 21-25. It should be appreciated that process 1900 may include any number of additional or alternative tasks, the tasks shown in FIG. 27 need not be performed in the illustrated order, and process 1900 may be incorporated into a more comprehensive procedure or process having additional functionality not described in detail here.

Process 1900 generally depends on data communication between the local medical devices and the network router device. Such data communication may be performed in an asynchronous or pseudorandomly scheduled manner or it may be performed on demand as needed. Process 1900 includes an optional task 1902 that represents an on-demand scenario where data from the medical devices is requested by the router device. During task 1902, the router device generates and transmits query messages to request data from the medical devices. In this example, each query message represents a request for an additional sensor measurement value (a glucose measurement value). Depending upon the system implementation and the current operating conditions, the query messages will be transmitted to the medical devices via a wired local data communication interface (such as wired local data communication interface 1708) or via a wireless local data communication interface (such as wireless local data communication interface 1712). The medical devices respond to the query messages by sending a current sensor measurement value. In practice, the current sensor measurement value may be sent along with a number of past measurements for redundancy.

Process 1900 eventually receives, from different medical devices, communication signals that convey the desired sensor measurement values generated by the respective sensor transceivers (task 1904). In this embodiment, the router device receives the glucose sensor measurement values via its local wired data communication interface or its local wireless data communication interface. Normally, the router device receives the sensor measurement values from the sensor transceivers. Alternatively, the router device may receive the sensor measurement values indirectly via a network appliance and the network architecture as described above in connection with FIG. 22. These sensor measurement values may be stored by the router device for later processing if so desired. Process 1900 assumes, however, that the router device processes the sensor measurement values as they arrive.

In example embodiments, a communication signal generated by an originating sensor transceiver conveys the corresponding device identifier. The network router device can then process the device identifiers for the various received signals in a suitable manner. For example, the router device may extract or process the device identifiers for the received data communication signals (task 1906), and process the sensor data conveyed in those data communication signals in a manner that is determined, governed, or dictated by the device identifiers. This technique enables the router device to identify the originating sensor transceiver, the originating patient, the sensor transceiver type, or other pertinent information. Process 1900 can then process, store, and/or route the received sensor data in an appropriate manner. As another example, the router device may receive a first communication signal from a first sensor transceiver, receive a second communication signal from a second sensor transceiver, obtain or extract the two respective device identifiers (which should be different), and process the sensor data conveyed in the two communication signals in a synchronized manner that is determined, governed, or dictated by the device identifiers. This technique enables the router device to prioritize the receipt, processing, storage, and/or transmission of sensor data depending upon the originating source.

In connection with the processing of raw sensor data, process 1900 may use the device identifiers to obtain the respective patient calibration values corresponding to each sensor measurement value and calculate calibrated BG levels using, for example, calibration algorithm(s) 1724. Thus, each calibrated BG level is calculated from (1) a calibration value derived from a glucose meter measurement, and (2) a raw glucose sensor measurement value. Process 1900 may then establish relationships or links between the various data items corresponding to the device identifiers. For example, each processed sensor measurement value can be linked to its device identifier, its patient, its calibrated blood glucose level, and other patient-related data (task 1910) for processing, formatting, and/or storage by the router device. Process 1900 may store the calibrated BG levels for the different patients (task 1912) for subsequent handling and report generation. In this regard, the router device may initiate such storage in its internal or attached memory, or it may initiate such storage in a network storage element coupled thereto. In practice, the calibrated BG levels for multiple patients can be stored in a common memory element that is formatted in an appropriate manner to enable centralized storage, retrieval, and processing.

As mentioned above, the network router device preferably includes an integrated or embedded web server application that allows the network router device to provide HTML documents to remote computing devices having suitably configured web browser applications. The router device can generate various management, setup, initialization, and configuration web pages, along with various web pages related to traditional medical device monitoring features and displays (as explained above in connection with the different monitor devices). In this regard, the processing architecture of the network router device is suitably configured to format the calibrated physiological characteristic measurement values for presentation in one or more HTML documents (task 1914). For this particular example, processing architecture 1702 may perform HTML formatting 1730 using conventional techniques to generate HTML web pages that contain calibrated BG levels. In accordance with known techniques and technologies, the router device and its web server application employ URLs that identify each web page formatted during task 1914.

Process 1900 can also be used to route, transmit, or otherwise communicate the calibrated BG levels to one or more network devices that are coupled to the network router device (task 1916). Depending upon the system implementation and the current operating conditions, the router device will communicate the calibrated BG levels via a wired network data communication interface (such as wired network data communication interface 1714) or via a wireless network data communication interface (such as wireless network data communication interface 1710). Thus, the router device can employ any suitable data communication protocol and transport mechanism to communicate data to other network components. For example, the router device may serve HTML documents containing the calibrated measurement values, transmit Ethernet data packets containing the calibrated measurement values, transmit 802.11 data packets containing the calibrated measurement values, or the like.

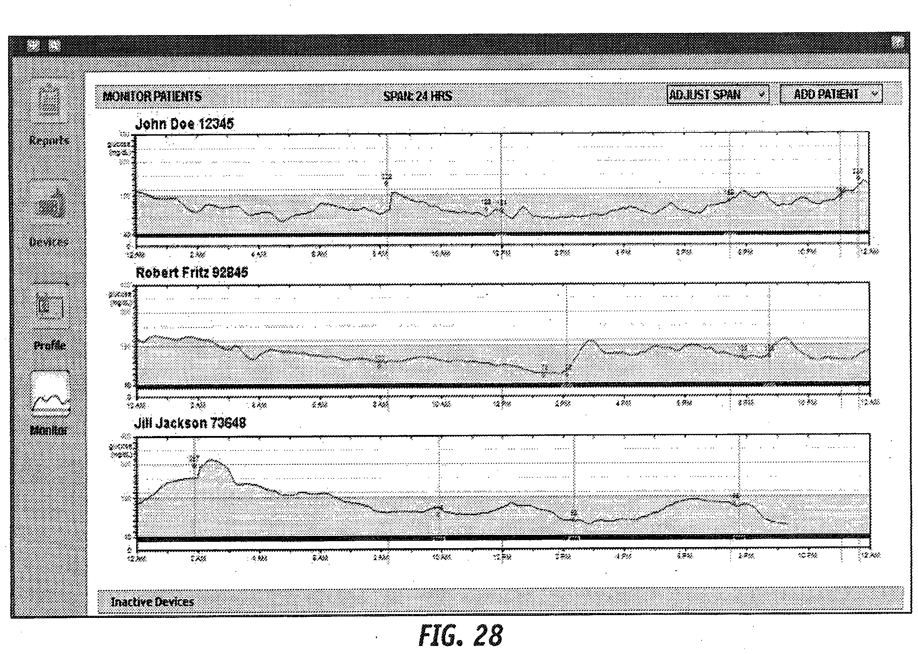

In preferred embodiments, the network router device utilizes its internal web server application to route suitably formatted web pages to requesting network computing devices. During task 1916, therefore, the network router device may receive a request for an HTML document that includes calibrated measurement value(s), where the request originates from a computing device that is in communication with the router device. For example, the request may be generated by a web browser application running on a computer device that is connected to the Internet; the request may include or indicate a desired URL corresponding to a requested web page. In response to the request, the router device (in particular, the web server application) will transmit the desired HTML document to the requesting computer device for presentation as a web page. The web browser application running on the requesting computer device generates the web page display in a conventional manner. For example, FIG. 28 is a sample screen shot of a patient monitor web page that might be generated by process 1900. Such a monitor web page is very useful in an environment, such as a hospital, where multiple patients will be monitored at the same time. Here, FIG. 28 illustrates time charts of calibrated BG levels for three different patients.

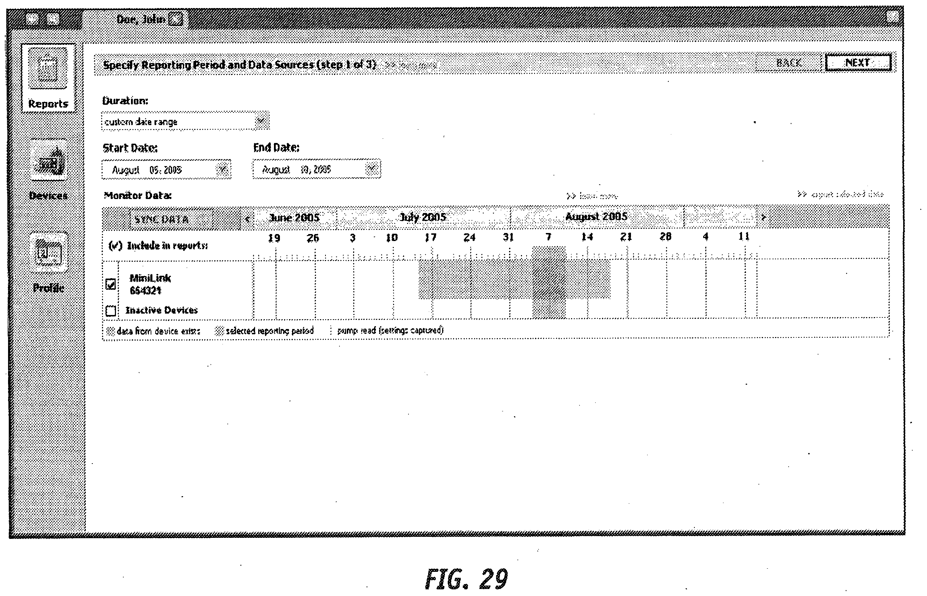

The network router device may also be configured to support additional data entries from patients and/or caregivers. For example, process 1900 may generate any number of data entry web pages that include additional patient data entry fields for: glucose readings from lab work; insulin dosages; carbohydrate intake; exercise levels and time periods; or the like. Moreover, the network router device may be configured to support other features and functions such as, without limitation: printing; reporting; graphing and charting; data exporting; emailing; or the like. For example, FIG. 29 is a sample screen shot of a patient reporting web page that might be generated by process 1900. This web page allows the patient (John Doe in this example) or caregiver to specify the date range and possibly other characteristics of the report. Here, FIG. 29 represents the first of three web pages related to the configuration of patient reports (the remaining two web pages are not shown).

Of course, the actual number of user interface screens, web pages, features, and functions associated with a network router device as described herein need not be limited to those described here, and a network router device can be suitably configured to support any number of desired operations.

While at least one example embodiment has been presented in the foregoing detailed description, it should be appreciated that a vast number of variations exist. It should also be appreciated that the example embodiment or embodiments described herein are not intended to limit the scope, applicability, or configuration of the invention in any way. Rather, the foregoing detailed description will provide those skilled in the art with a convenient road map for implementing the described embodiment or embodiments. It should be understood that various changes can be made in the function and arrangement of elements without departing from the scope of the invention, where the scope of the invention is defined by the claims, which includes known equivalents and foreseeable equivalents at the time of filing this patent application.

## Claims

1. A communication method for a wireless telemetry router device (1500), the method comprising:
receiving, at the wireless telemetry router device (1500), a plurality of wireless communication signals, each of the wireless communication signals conveying sensor data comprising raw data generated by a respective continuous glucose sensor;
wherein each of the wireless communication signals conveys a sensor identifier corresponding to a respective one of the sensors (1502), wherein the sensor identifier is the serial number of the sensor;
obtaining the sensor identifiers from the wireless communication signals; and
obtaining, at the wireless telemetry router device, patient calibration values (1738) corresponding to respective continuous glucose sensors, based upon respective blood glucose measurements;
processing the sensor data conveyed in the wireless communication signals in a manner determined by the sensor identifiers comprising calculating (1908), at the wireless telemetry router device (1500), respective calibrated blood glucose levels from the respective raw data and corresponding patient calibration values (1738);
generating a network communication in compliance with a network data communication protocol, the network dommuncation conveying at least some of the sensor data comprising the calibrated blood glucose levels; and
transmitting, in accordance with the network data communication protocol, the network communication to a network device (1508, 1510, 1512, 1514, 1516).

2. A method according to claim 1, further comprising storing at least some of the sensor data at the wireless telemetry router device (1500).

3. A method according to claim 1, wherein the transmitting step comprises transmitting the network communication in compliance with an Ethernet protocol.

4. A method according to claim 1, wherein the transmitting step comprises transmitting the network communication in compliance with an IEEE 802.11 protocol.

5. A method according to claim 1, wherein the transmitting step comprises transmitting the network communication in compliance with a Bluetooth protocol.

6. A method according to claim 1, further comprising reformatting data conveyed in the wireless communication signals for compatibility with the network data communication protocol.

7. A network router device (1700) for centralized processing of medical device data, the network router device (1700) comprising:
a first data communication interface (1712) configured to receive a plurality of wireless communication signals, each of the wireless communication signals conveying a sensor measurement value as raw data generated by a respective continuous glucose sensor;
a processing architecture (1702) coupled to the first data communication interface (1712), the processing architecture (1702) being configured to generate network communications in compliance with a network data communication protocol,
the network communications conveying calibrated blood glucose levels; and
a second data communication interface (1710, 1714) coupled to the processing architecture (1702), the second data communication interface (1710, 1714) being configured to transmit, in accordance with the network data communication protocol, the network communications to at least one network device;
wherein each of the wireless communication signals conveys a sensor identifier corresponding to a respective one of the sensors (1502), wherein the sensor identifier is the serial number of the sensor;
the processing architectire being further configured to:
obtain the sensor identifiers in the received wireless communication signals;
obtain at the router device stored patient calibration values (1738) corresponding to the respective sensors, based upon respective blood glucose measurements; and
process the sensor data conveyed in the received wireless communication signals in a manner determined by the respective sensor identifiers comprising calculating each calibrated blood glucose level from the respective raw data and corresponding patient calibration value.

8. A network router device (1700) according to claim 7. further compriding a memory element (1704) configured to store at least some of the calibrated blood glucose levels.

9. A network router device (1700) according to claim 7, wherein the second data communication interface (1710, 1714) is compliant with an Ethernet protocol, or with a wireless data communication protocol used by the continuous glucose sensors.

10. A network router device (1700) according to claim 7, wherein the processing architecture (1702) is configured to format the calibrated blood glucose levels for presentation in an HTML document.

## Patentansprüche

1. Kommunikationsverfahren für ein drahtloses Telemetrie-Routergerät (1500), wobei das Verfahren Folgendes umfasst:
das Empfangen einer Vielzahl von Drahtlos-Kommunikationssignalen am Drahtlos-Telemetrie-Routergerät (1500), wobei jedes der Drahtlos-Kommunikationssignale Sensordaten überträgt,
die Rohdaten umfassen, die von einem jeweiligen kontinuierlich arbeitenden Blutzuckersensor erzeugt werden;
wobei jedes der Drahtlos-Kommunikationssignale eine Sensorkennung entsprechend einem der jeweiligen Sensoren (1502) überträgt, wobei die Sensorkennung die Seriennummer des Sensors ist;
das Gewinnen der Sensorkennungen aus den Drahtlos-Kommunikationssignalen; und das Gewinnen von Patientenkalibrierungswerten (1738) entsprechend den jeweiligen kontinuierlich arbeitenden Blutzuckersensoren an dem Drahtlos-Telemetrie-Routergerät, die Blutzuckermessungen entsprechen;
das Verarbeiten der in den Drahtlos-Kommunikationssignalen übertragenen Sensordaten auf eine von den Sensorkennern festgelegte Weise, umfassend das Berechnen (1908) der entsprechenden kalibrierten Blutzuckerspiegel aus den jeweiligen Rohdaten und den entsprechenden Patientenkalibrierungswerten (1738) an dem Drahtlos-Telemetrie-Routergerät (1500);
das Erzeugen einer Netzwerk-Kommunikation entsprechend einem Netzwerk-Datenkommunikationsprotokoll, wobei mittels der Netzwerk-Kommunikation mindestens einige der Sensordaten, die die kalibrierten Blutzuckerspiegel umfassen, übertragen werden; und
das Senden der Netzwerk-Kommunikation entsprechend dem Netzwerk-Datenkommunikationsprotokoll an ein Netzwerkgerät (1508,1510,1512,1514,1516).

2. Verfahren nach Anspruch 1, das ferner das Speichern mindestens einiger der Sensordaten an dem Drahtlos-Telemetrie-Routergerät (1500) umfasst.

3. Verfahren nach Anspruch 1, bei dem der Sendeschritt das Senden der Netzwerk-Kommunikation entsprechend einem Ethernet-Protokoll umfasst.

4. Verfahren nach Anspruch 1, bei dem der Sendeschritt das Senden der Netzwerk-Kommunikation entsprechend einem IEEE 802.11-Protokoll umfasst.

5. Verfahren nach Anspruch 1, bei dem der Sendeschritt das Senden der Netzwerk-Kommunikation entsprechend einem Bluetooth-Protokoll umfasst.

6. Verfahren nach Anspruch 1, das ferner das Umformatieren der in den Drahtlos-Kommunikationssignalen übertragenen Daten für die Kompatibilität mit dem Netzwerk-Datenkommunikationsprotokoll umfasst.

7. Netzwerk-Routergerät (1700) zur zentralisierten Verarbeitung von Daten medizinischer Geräte, wobei das
Netzwerk-Routergerät (1700) Folgendes umfasst:
eine erste Daten-Kommunikationsschnittstelle (1712), die dazu konfiguriert ist, eine Vielzahl von Drahtlos-Kommunikationssignalen zu empfangen, die jeweils einen Sensor-Messwert als Rohdaten, die von einem jeweiligen kontinuierlich arbeitenden Blutzuckersensor erzeugt werden, übertragen;
eine Verarbeitungsarchitektur (1702) die mit der ersten Daten-Kommunikationsschnittstelle (1712) gekoppelt ist, wobei die Verarbeitungsarchitektur (1702) dafür konfiguriert ist, Netzwerk-Kommunikation entsprechend einem Netzwerk-Datenkommunikationsprotokoll zu erzeugen,
wobei die Netzwerk-Kommunikation kalibrierte Blutzuckerspiegel überträgt; und eine zweite Daten-Kommunikationsschnittstelle (1710, 1714), die mit der Verarbeitungsarchitektur (1702) gekoppelt ist, wobei die zweite Daten-Kommunikationsschnittstelle (1710,1714) dafür konfiguriert ist, die Netzwerkkommunikation entsprechend dem Netzwerk-Datenkommunikationsprotokoll an mindestens ein Netzwerkgerät zu senden;
wobei jedes der Drahtlos-Kommunikationssignale eine Sensorkennung entsprechend einem der jeweiligen Sensoren (1502) überträgt, wobei die Sensorkennung die Seriennummer des Sensors ist;
wobei die Verarbeitungsarchitektur ferner für Folgendes konfiguriert ist:
das Erhalten der Sensorkennungen in den empfangenen Drahtlos-Kommunikationssignalen;
das Erhalten von gespeicherten Patientenakalibrierungswerten (1738) entsprechend den jeweiligen Sensoren an dem Routergerät, die auf entsprechenden Blutzuckermessungen basieren; und
das Verarbeiten der in den empfangenen Drahtlos-Kommunikationssignalen übertragenen Sensordaten auf eine von den jeweiligen Sensorkennungen festgelegte Weise, umfassend das Berechnen jedes kalibrierten Blutzuckerspiegels aus den jeweiligen Rohdaten und dem entsprechenden Patientenkalibrierungswert.

8. Netzwerk-Routergerät (1700) nach Anspruch 7, das ferner ein Speicherelement (1704) umfasst, das dafür konfiguriert ist, mindestens einige der kalibrierten Blutzuckerspiegel zu speichern.

9. Netzwerk-Routergerät (1700) nach Anspruch 7, wobei die zweite Daten-Kommunikationsschnittstelle (1710,1714) mit einem Ethernet-Protokoll oder mit einem Drahtlos-Datenkommunikationsprotokoll, das von den kontinuierlich arbeitenden Blutzuckersensoren verwendet wird, konform ist.

10. Netzwerk-Routergerät (1700) nach Anspruch 7, wobei die Verarbeitungsarchitektur (1702) dafür konfiguriert ist, dass sie die kalibrierten Blutzuckerspiegel zur Darstellung in einem HTML-Dokument formatiert.

## Revendications

1. Procédé de communication pour un dispositif routeur de télémétrie sans fil (1500), le procédé comprenant :
la réception, au niveau du dispositif routeur de télémétrie sans fil (1500), d'une pluralité de signaux de communication sans fil, chacun des signaux de communication sans fil transportant des données de capteur comprenant des données brutes générées par un capteur de glucose en continu respectif ;
dans lequel chacun des signaux de communication sans fil transporte un identificateur de capteur correspondant à l'un respectif des capteurs (1502), dans lequel l'identificateur de capteur est le numéro de série du capteur ;
l'obtention des identificateurs de capteur à partir des signaux de communication sans fil ; et l'obtention, au niveau du dispositif routeur de télémétrie sans fil, de valeurs d'étalonnage de patient (1738) correspondant à des capteurs de glucose en continu respectifs, sur la base de mesures de glycémie respectives ;
le traitement des données de capteur transportées dans les signaux de communication sans fil d'une manière déterminée par les identificateurs de capteur comprenant le calcul (1908), au niveau du dispositif routeur de télémétrie sans fil (1500), de taux de glycémie étalonnés respectifs à partir des données brutes respectives et de valeurs d'étalonnage de patient correspondantes (1738) ;
la génération d'une communication réseau en conformité avec un protocole de communication de données réseau, la communication réseau transportant au moins certaines des données de capteur comprenant les taux de glycémie étalonnés ; et
la transmission, conformément au protocole de communication de données réseau, de la communication réseau à un dispositif réseau (1508, 1510, 1512, 1514, 1516).

2. Procédé selon la revendication 1, comprenant en outre le stockage d'au moins certaines des données de capteur au niveau du dispositif routeur de télémétrie sans fil (1500).

3. Procédé selon la revendication 1, dans lequel l'étape de transmission comprend la transmission de la communication réseau en conformité avec un protocole Ethernet.

4. Procédé selon la revendication 1, dans lequel l'étape de transmission comprend la transmission de la communication réseau en conformité avec un protocole IEEE 802.11.

5. Procédé selon la revendication 1, dans lequel l'étape de transmission comprend la transmission de la communication réseau en conformité avec un protocole Bluetooth.

6. Procédé selon la revendication 1, comprenant en outre le reformatage des données transportées dans les signaux de communication sans fil pour une compatibilité avec le protocole de communication de données réseau.

7. Dispositif routeur réseau (1700) pour un traitement centralisé de données de dispositif médical, le dispositif routeur réseau (1700) comprenant :
une première interface de communication de données (1712) configurée pour recevoir une pluralité de signaux de communication sans fil, chacun des signaux de communication sans fil transportant une valeur de mesure de capteur en tant que données brutes générées par un capteur de glucose en continu respectif ;
une architecture de traitement (1702) couplée à la première interface de communication de données (1712), l'architecture de traitement (1702) étant configurée pour générer des communications réseau en conformité avec un protocole de communication de données réseau,
les communications réseau transportant des taux de glycémie étalonnés ; et
une deuxième interface de communication de données (1710, 1714) couplée à l'architecture de traitement (1702), la deuxième interface de communication de données (1710, 1714) étant configurée pour transmettre, conformément au protocole de communication de données réseau, les communications réseau vers au moins un dispositif réseau ;
dans lequel chacun des signaux de communication sans fil transporte un identificateur de capteur correspondant à l'un respectif des capteurs (1502), dans lequel l'identificateur de capteur est le numéro de série du capteur ;
l'architecture de traitement étant configurée en outre pour :
obtenir les identificateurs de capteur dans les signaux de communication sans fil reçus ;
obtenir au niveau du dispositif routeur des valeurs d'étalonnage de patient stockées (1738) correspondant aux capteurs respectifs, sur la base de mesures de glycémie respectives ; et
traiter les données de capteur transportées dans les signaux de communication sans fil reçus d'une manière déterminée par les identificateurs de capteur respectifs comprenant le calcul de chaque taux de glycémie étalonné à partir des données brutes respectives et de la valeur d'étalonnage de patient correspondante.

8. Dispositif routeur réseau (1700) selon la revendication 7, comprenant en outre un élément de mémoire (1704) configuré pour stocker au moins certains des taux de glycémie étalonnés.

9. Dispositif routeur réseau (1700) selon la revendication 7, dans lequel la deuxième interface de communication de données (1710, 1714) est conforme à un protocole Ethernet, ou à un protocole de communication de données sans fil utilisé par les capteurs de glucose en continu.

10. Dispositif routeur réseau (1700) selon la revendication 7, dans lequel l'architecture de traitement (1702) est configurée pour formater les taux de glycémie étalonnés pour présentation dans un document HTML.
